(19) Europäisches Patentamt / European Patent Office / Office européen des brevets

(11) **EP 4 390 455 A1**

(12) **EUROPEAN PATENT APPLICATION**
published in accordance with Art. 153(4) EPC

(43) Date of publication:
**26.06.2024 Bulletin 2024/26**

(21) Application number: **22858319.1**

(22) Date of filing: **03.08.2022**

(51) International Patent Classification (IPC):
**G01S 13/34** $^{(2006.01)}$    **A61B 5/0245** $^{(2006.01)}$

(52) Cooperative Patent Classification (CPC):
**A61B 5/02; A61B 5/0245; A61B 5/11; G01S 13/34; G01S 13/58**

(86) International application number:
**PCT/JP2022/029856**

(87) International publication number:
**WO 2023/021998 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.08.2021 JP 2021132423**

(71) Applicant: **Kyocera Corporation**
**Kyoto-shi Kyoto 612-8501 (JP)**

(72) Inventors:
• **KURODA, Jun**
  **Kyoto-shi, Kyoto 612-8501 (JP)**

• **SAHARA, Tooru**
  **Kyoto-shi, Kyoto 612-8501 (JP)**
• **YAMAMOTO, Kenji**
  **Kyoto-shi, Kyoto 612-8501 (JP)**
• **HOMMA, Takuya**
  **Kyoto-shi, Kyoto 612-8501 (JP)**
• **TONG, Fangwei**
  **Kyoto-shi, Kyoto 612-8501 (JP)**

(74) Representative: **Viering, Jentschura & Partner mbB**
**Patent- und Rechtsanwälte**
**Am Brauhaus 8**
**01099 Dresden (DE)**

(54) **ELECTRONIC DEVICE, METHOD FOR CONTROLLING ELECTRONIC DEVICE, AND PROGRAM**

(57)      An electronic device includes a transmission antenna, a reception antenna, and a signal processor. The transmission antenna transmits a transmission wave. The reception antenna receives a reflected wave that is the transmission wave having been reflected. Based on a transmission signal transmitted as the transmission wave and a reception signal received as the reflected wave, the signal processor detects oscillations arising from a heartbeat of a subject that reflects the transmission wave. Based on oscillation velocity obtained through oscillation component removal except for an oscillation component lying at a desired position from a result of performing a fast Fourier transform in a range direction and a velocity direction on the reception signal, the signal processor calculates the heartbeat of the subject.

FIG. 2

EP 4 390 455 A1

**Description**

CROSS-REFERENCE TO RELATED APPLICATIONS

This application claims priority from Japanese Patent Application No. 2021-132423 filed in Japan on August 16, 2021, the entire disclosure of which is incorporated herein by reference.

TECHNICAL FIELD

The present disclosure relates to an electronic device, a method for controlling an electronic device, and a program.

BACKGROUND OF INVENTION

For example, in fields of automobile-related industries and the like, a technology for measuring a distance between a vehicle of interest and a predetermined object, and the like, is regarded as important. More particularly, various studies have recently been conducted on a radar (Radio Detecting and Ranging) technology for measuring a distance, etc. to an object such as an obstacle by transmitting a radio wave such as a millimeter wave and then receiving a wave reflected off the object. The importance of such a technology for measuring a distance, etc. is expected to grow more and more in the future with progresses of technologies for assisting drivers in driving and autonomous-driving-related technologies for partially or entirely automating driving.

Various suggestions have been made regarding a technology for detecting the presence, etc. of a predetermined object by receiving a reflected wave coming back from the object due to reflection of a transmitted radio wave. For example, Patent Literature 1 has proposed a device capable of detecting the presence of a person and biological information on the person by utilizing a microwave. As another example, Patent Literature 2 has proposed an apparatus for detecting a vital sign such as a frequency of respiration of a living body or a heartbeat thereof on the basis of a reflected signal of a microwave radar.

CITATION LIST

PATENT LITERATURE

Patent Literature 1: Japanese Unexamined Patent Application Publication No. 2002-71825
Patent Literature 2: Japanese Unexamined Patent Application Publication No. 2021-32880

SUMMARY

In one embodiment, an electronic device includes a transmission antenna, a reception antenna, and a signal processor. The transmission antenna transmits a transmission wave. The reception antenna receives a reflected wave, the reflected wave being the transmission wave having been reflected. Based on a transmission signal transmitted as the transmission wave and a reception signal received as the reflected wave, the signal processor detects oscillations arising from a heartbeat of a subject that reflects the transmission wave.

Based on oscillation velocity obtained through oscillation component removal except for an oscillation component lying at a desired position from a result of performing a fast Fourier transform in a range direction and a velocity direction on the reception signal, the signal processor calculates the heartbeat of the subject.

In one embodiment, an electronic device is configured to detect, based on a transmission signal transmitted as a transmission wave from a transmission antenna and a reception signal received via a reception antenna as a reflected wave, the reflected wave being the transmission wave having been reflected, oscillations arising from a heartbeat of a subject that reflects the transmission wave.

Based on oscillation velocity obtained through oscillation component removal except for an oscillation component lying at a desired position from a result of performing a fast Fourier transform in a range direction and a velocity direction on the reception signal, the electronic device calculates the heartbeat of the subject.

In one embodiment, a method for controlling an electronic device includes transmission, reception, detection, and calculation.

The transmission transmits a transmission wave from a transmission antenna.

The reception receives, via a reception antenna, a reflected wave, the reflected wave being the transmission wave having been reflected.

The detection detects, based on a transmission signal transmitted as the transmission wave and a reception signal received as the reflected wave, oscillations arising from a heartbeat of a subject that reflects the transmission wave.

The calculation calculates the heartbeat of the subject, based on oscillation velocity obtained through oscillation component removal except for an oscillation component lying at a desired position from a result of performing a fast Fourier transform in a range direction and a velocity direction on the reception signal.

In one embodiment, a program causes an electronic device to execute transmission, reception, detection, and calculation.

The transmission transmits a transmission wave from a transmission antenna.

The reception receives, via a reception antenna, a reflected wave, the reflected wave being the transmission wave having been reflected.

The detection detects, based on a transmission signal transmitted as the transmission wave and a reception signal received as the reflected wave, oscillations arising from a heartbeat of a subject that reflects the transmission wave.

The calculation calculates the heartbeat of the subject, based on oscillation velocity obtained through oscillation component removal except for an oscillation component lying at a desired position from a result of performing a fast Fourier transform in a range direction and a velocity direction on the reception signal.

BRIEF DESCRIPTION OF THE DRAWINGS

FIG. 1 is a diagram for explaining how an electronic device according to one embodiment is used.

FIG. 2 is a functional block diagram schematically illustrating a configuration of the electronic device according to one embodiment.

FIG. 3 is a diagram for explaining a configuration of a signal processed by the electronic device according to one embodiment.

FIG. 4 is a diagram for explaining processing of a signal by the electronic device according to one embodiment.

FIG. 5 is a diagram for explaining processing of a signal by the electronic device according to one embodiment.

FIG. 6 is a diagram for explaining processing of a signal by the electronic device according to one embodiment.

FIG. 7 is a diagram schematically illustrating an example of arrangement of antennas in an antenna array of the electronic device according to one embodiment, and an operation principle thereof.

FIG. 8 is a diagram illustrating an example of arrangement of the antennas in the antenna array of the electronic device according to one embodiment.

FIG. 9 is a diagram illustrating an example of processing of a signal by the electronic device according to one embodiment.

FIG. 10 is a diagram illustrating an example of processing of a signal by the electronic device according to one embodiment.

FIG. 11 is a flowchart illustrating a comparative example of operation of the electronic device according to one embodiment.

FIG. 12 is a flowchart for explaining operation of the electronic device according to one embodiment.

FIG. 13 is a flowchart for explaining operation of the electronic device according to one embodiment.

FIG. 14 is a diagram illustrating an example of processing of a signal by the electronic device according to one embodiment.

FIG. 15 is a diagram for explaining an example of processing of a signal by the electronic device according to one embodiment.

FIG. 16 is a diagram illustrating an example of processing of a signal by the electronic device according to one embodiment.

FIG. 17 is a diagram illustrating an example of processing of a signal by the electronic device according to one embodiment.

FIG. 18 is a diagram illustrating an example of processing of a signal by the electronic device according to one embodiment.

FIG. 19 is a diagram illustrating an example of processing of a signal by the electronic device according to one embodiment.

FIG. 20 is a diagram illustrating an example of processing of a signal by the electronic device according to one embodiment.

FIG. 21 is a diagram illustrating an example of processing of a signal by the electronic device according to one embodiment.

FIG. 22 is a diagram illustrating an example of processing of a signal by the electronic device according to one embodiment.

FIG. 23 is a diagram illustrating an example of arrangement of the antennas in the antenna array of the electronic device according to one embodiment.

DESCRIPTION OF EMBODIMENTS

Usefulness in various fields can be expected if weak oscillations such as those of a heartbeat in a human body or the like can be detected with good accuracy through transmission and reception of a radio wave such as, for example, a millimeter wave. The present disclosure provides an electronic device capable of detecting a heartbeat in a human body or the like with good accuracy through transmission and reception of a radio wave, a method for controlling an electronic device, and a program. One embodiment makes it possible to provide an electronic device capable of detecting a heartbeat in a human body or the like with good accuracy through transmission and reception of a radio wave, a method for controlling an electronic device, and a program. One embodiment will now be described in detail with reference to the drawings.

In the present disclosure, the term "electronic device" may refer to a device driven by electric power. The term "user" may refer to an entity (typically, a human) using, or an animal using, a system and/or an electronic device according to one embodiment. The term "user" may encompass an entity who monitors a subject such as a human by using the electronic device according to one embodiment. The term "subject" may refer to an entity (for example, a human or an animal) to be monitored with the electronic device according to one embodiment. The term "user" may encompass the subject.

The electronic device according to one embodiment is capable of detecting a heartbeat of a subject such as a human located in the neighborhood of the electronic device. Accordingly, expected scenes where the electronic device according to one embodiment will be used may be specific facilities used by entities who perform social activities, such as, for example, a company, a hospital, a nursing home, a school, a gym, and a care facility. For example, in the case of a company, grasping and/or managing health conditions of employees and the like are very important. Likewise, in the case of a hospital, grasping and/or managing health conditions of patients, healthcare workers, and the like are very important. In the case of a nursing home, grasping and/or managing health conditions of residents, staff members, and the like are very important. The scenes where the electronic device according to one embodiment will be used are not limited to the above-mentioned facilities such as a company, a hospital, and a nursing home, but may be any facility where grasping and/or managing health conditions of a subject are demanded. "Any facility" may include non-commercial facilities such as a house of a user. The scenes where the electronic device according to one embodiment is used are not limited to indoor places, but may be outdoor places. For example, the scenes where the electronic device according to one embodiment will be used may be the inside of mobility means such as a train, a bus, and an airplane, a station, a landing, and the like. The electronic device according to one embodiment may be used aboard mobility means such as an automobile, an aircraft, or a vessel, at a hotel, at a house of a user, in a living room of the user's house, a bathroom thereof, a lavatory thereof, a bedroom thereof, or the like.

For example, the electronic device according to one embodiment may be used for the purpose of detecting or monitoring a heartbeat of a subject such as a person requiring medical care or a person requiring nursing care at a care facility or the like. Upon finding something abnormal in the heartbeat of the subject such as a person requiring medical care or a person requiring nursing care, for example, the electronic device according to one embodiment may issue a predetermined warning to, for example, the subject himself/herself and/or another person. Thus, the electronic device according to one embodiment enables the subject such as, for example, a person requiring medical care or a person requiring nursing care, and/or a staff member at a care facility or the like, to grasp the abnormality in the pulse of the subject. Upon finding nothing abnormal in the heartbeat of the subject such as a person requiring medical care or a person requiring nursing care, for example, the electronic device according to one embodiment may inform, for example, the subject himself/herself and/or another person that no abnormality is found in the heartbeat. Thus, the electronic device according to one embodiment enables the subject such as, for example, a person requiring medical care or a person requiring nursing care, and/or a staff member at a care facility or the like, to grasp that the pulse of the subject is normal.

The electronic device according to one embodiment may detect the pulse of a subject other than a human, such as an animal. The description will be given below on an assumption that the electronic device according to one embodiment detects the pulse of a human by means of a sensor based on a technology such as a millimeter-wave radar, for example.

The electronic device according to one embodiment may be installed in or on any stationary object or may be installed in or on any mobility device. The electronic device according to one embodiment is capable of transmitting a transmission wave to an area around the electronic device from a transmission antenna. The electronic device according to one embodiment is capable of receiving, by means of a reception antenna, a reflected wave, the reflected wave being the transmission wave having been reflected. The electronic device may include at least one of the transmission antenna or the reception antenna. Alternatively, for example, a radar sensor or the like may include at least one of the transmission antenna or the reception antenna.

In the description given below, as a typical example, the electronic device according to one embodiment is assumed to be stationary. On the other hand, a subject (a human) whose pulse is to be detected by the electronic device according to one embodiment may be stationary, may be moving, or may be moving his/her body while being stationary. As is the case with an ordinary radar sensor, the electronic device according to one embodiment is capable of measuring a distance, etc. between the electronic device and an object located in the neighborhood of the electronic device when the object is movable. The electronic device according to one embodiment is capable of measuring a distance, etc. between the electronic device and the object even if both the electronic device and the object are stationary.

The electronic device according to one embodiment will be described in detail below with reference to the drawings. First, an example of detecting an object by the electronic device according to one embodiment will now be described.

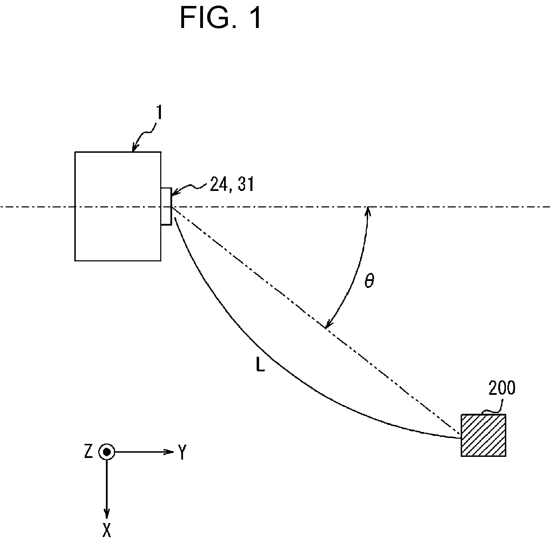

FIG. 1 is a diagram for explaining an example of how the electronic device according to one embodiment is used. FIG. 1 illustrates an example of the electronic device according to one embodiment that has functions of a sensor including a transmission antenna and a reception antenna.

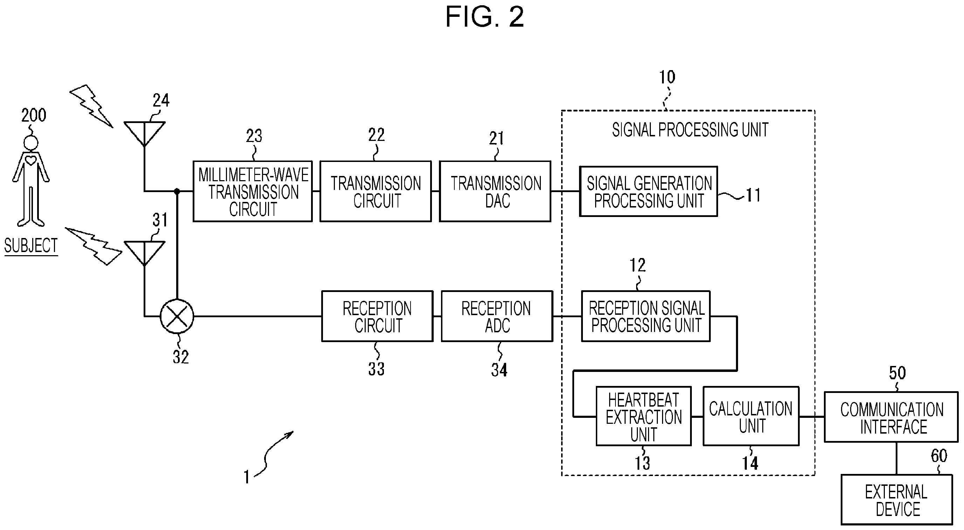

As illustrated in FIG. 1, an electronic device 1 according to one embodiment may include a transmission unit and a reception unit that will be described later. As will be described later, the transmission unit may include a transmission antenna array 24. The reception unit may include a reception antenna array 31. Specific configurations of the electronic device 1, the transmission unit, and the reception unit will be described later. FIG. 1 illustrates a circumstance in which, for easier view, the electronic device 1 includes the transmission antenna array 24 and the reception antenna array 31. The electronic device 1 may include, as appropriate, at least any of other functionalities, such as at least a part of a signal processing unit 10 (FIG. 2) included in the electronic device 1. The electronic device 1 may be configured such that at least any of other functionalities, such as at least a part of the signal processing unit 10 (FIG. 2) included in the electronic device 1, is provided outside the electronic device 1. In FIG. 1, the electronic device 1 may be moving, or be stationary without moving.

The example illustrated in FIG. 1 gives a simplified view of the transmission unit including the transmission antenna array 24 and the reception unit including the reception antenna array 31 of the electronic device 1. The electronic device 1 may include a plurality of transmission units and a plurality of reception units. The transmission unit may include the transmission antenna array 24 made up of a plurality of transmission antennas. The reception unit may include the reception antenna array 31 made up of a plurality of reception antennas. A position where the transmission unit and/or the reception unit is/are installed in or on the electronic device 1 is not limited to the position illustrated in FIG. 1 but may be any other position. The number of transmission units and/or the number of reception units may be any number equal to or greater than one, depending on various conditions (or requirements) such as a heartbeat detection range and/or a heartbeat detection accuracy to be achieved by the electronic device 1.

As will be described later, the electronic device 1 transmits an electromagnetic wave as a transmission wave from the transmission antenna array 24. For example, when a predetermined object entity (for example, a subject 200 illustrated in FIG. 1) is located in the neighborhood of the electronic device 1, at least a part of the transmission wave transmitted from the electronic device 1 is reflected off the object entity to turn into a reflected wave. Then, for example, the reception antenna array 31 of the electronic device 1 receives such a reflected wave. By this means, the electronic device 1 can detect the subject as a target.

The electronic device 1 including the transmission antenna array 24 may be typically a radar (Radio Detecting and Ranging) sensor configured to transmit and receive a radio wave. However, the electronic device 1 is not limited to a radar sensor. The electronic device 1 according to one embodiment may be, for example, a sensor based on the LIDAR (Light Detection and Ranging, Laser Imaging Detection and Ranging) technology that uses an optical wave. These sensors can include, for example, patch antennas, etc. Since the RADAR and the LIDAR are known technologies, detailed description thereof will be sometimes omitted, or the description thereof will be sometimes simplified, where appropriate. The electronic device 1 according to one embodiment may be a sensor that is based on a technology of detecting an object entity by transmitting and receiving a sound wave or an ultrasound wave, for example.

The electronic device 1 illustrated in FIG. 1 receives, via the reception antenna array 31, the reflected wave coming back due to reflection of the transmission wave transmitted from the transmission antenna array 24. In this way, the electronic device 1 can detect, as the target, the predetermined subject 200 located within a predetermined distance from the electronic device 1. For example, as illustrated in FIG. 1, the electronic device 1 is capable of measuring a distance L between the electronic device 1 and the predetermined subject 200. The electronic device 1 is capable of measuring a relative velocity between the electronic device 1 and the predetermined subject 200. The electronic device 1 is capable of measuring a direction (an angle of arrival $\theta$) in which the reflected wave coming from the predetermined subject 200 arrives at the electronic device 1.

In FIG. 1, an XY plane may be, for example, a plane substantially parallel to a ground surface. In this case, a positive Z-axis direction illustrated in FIG. 1 may indicate a vertically upward direction. In FIG. 1, the electronic device 1 may be disposed on a plane parallel to the XY plane. In FIG. 1, the subject 200 may be in a state of, for example, standing on the ground surface substantially parallel to the XY plane.

The subject 200 may be, for example, a human or the like located in the neighborhood of the electronic device 1. Alternatively, the subject 200 may be, for example, a living thing other than a human, such as an animal located in the neighborhood of the electronic device 1. As described above, the subject 200 may be moving, stopped, or stationary. In the present disclosure, an object entity to be detected by the electronic device 1 includes living things such as a person, a dog, a cat, a horse, and other kinds of animal in addition to non-living things such as any object. The object entity to be detected by the electronic device 1 in the present disclosure may include a radar target including a person, an object, and an animal, etc. to be detected using the radar technology. In the description given below, an object entity such as the subject 200 located in the neighborhood of the electronic device 1 is assumed to be a human (or an animal). In the description given below, the "subject 200" will be referred to also as the "person 200 who is the subject" where appropriate.

In FIG. 1, a ratio between a size of the electronic device 1 and a size of the subject 200 does not necessarily indicate an actual ratio. FIG. 1 illustrates a state in which the transmission antenna array 24 of the transmission unit and the reception antenna array 31 of the reception unit are installed on an outer portion of the electronic device 1. However, in one embodiment, the transmission antenna array 24 of the transmission unit and/or the reception antenna array 31 of the reception unit may be installed at various positions of the electronic device 1. For example, in one embodiment, the transmission antenna array 24 of the transmission unit and/or the reception antenna array 31 of the reception unit may be installed inside the electronic device 1 so as not to appear on the exterior of the electronic device 1.

In the description given below, as a typical example, the transmission antenna of the electronic device 1 is assumed to transmit a radio wave in a frequency band, such as a millimeter wave (equal to or higher than 30 GHz) or a quasi-millimeter wave (for example, around 20 GHz to 30 GHz). On the other hand, the transmission antenna of the electronic device 1 may transmit a radio wave having a frequency bandwidth of 4 GHz such as, for example, from 77 GHz to 81 GHz.

FIG. 2 is a functional block diagram schematically illustrating an example of a configuration of the electronic device 1 according to one embodiment. An example of the configuration of the electronic device 1 according to one embodiment will be described below.

When a distance or the like is measured by using a millimeter-wave radar, a frequency modulated continuous wave radar (hereinafter abbreviated as "FM-CW radar") is often used. The FM-CW radar sweeps a frequency of a to-be-transmitted radio wave to generate a transmission signal. Therefore, the frequency of a radio wave used by, for example, a 79-GHz millimeter-wave FM-CW radar has a frequency bandwidth of 4 GHz such as from, for example, 77 GHz to 81 GHz. The radar in the frequency band of 79 GHz has a feature of a wider usable frequency bandwidth, as compared with other millimeter-wave/quasi-millimeter-wave radars in frequency bands of, for example, 24 GHz, 60 GHz, 76 GHz, and the like. Such an embodiment will be described below as an example.

The FM-CW radar scheme used in the present disclosure may include an FCM (Fast-Chirp Modulation) scheme, in which chirp signals are transmitted in a shorter cycle than usual. The signal generated by the electronic device 1 is not limited to a signal of the FM-CW scheme. The signal generated by the electronic device 1 may be a signal of various schemes other than the FM-CW scheme. A transmission signal sequence stored in any storage unit may differ from one to another of these various schemes. For example, in the case of a radar signal of the FM-CW scheme described above, a signal whose frequency increases/decreases for each time sample may be used. A more detailed description of the various schemes described above is omitted because known techniques can be employed as appropriate.

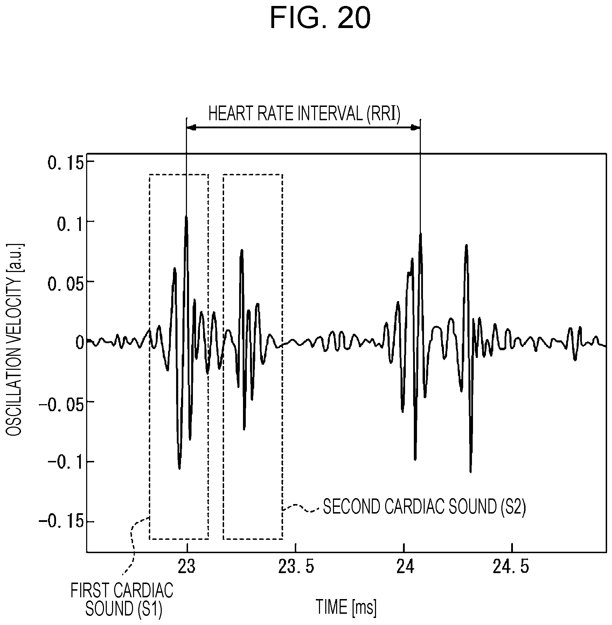

As illustrated in FIG. 2, the electronic device 1 according to one embodiment includes the signal processing unit 10. The signal processing unit 10 may include a signal generation processing unit 11, a reception signal processing unit 12, a heartbeat extraction unit 13, and a calculation unit 14. The heartbeat extraction unit 13 may perform processing of, for example, extracting micro-Doppler components. The heartbeat extraction unit 13 may perform processing of extracting an envelope of cardiac sound of the person 200 who is the subject. The calculation unit 14 may perform processing of, for example, extracting a heart rate interval (RRI) of the person 200 who is the subject. The calculation unit 14 may perform processing for frequency analysis of time-series data of the extracted heart rate interval of the person 200 who is the subject. Based on the frequency analysis of the time-series data of the heart rate interval, the calculation unit 14 may perform processing of calculating a heart rate variability of the person 200 who is the subject. A more detailed description of the signal generation processing unit 11, the reception signal processing unit 12, the heartbeat extraction unit 13, and the calculation unit 14 will be given later where appropriate. In the present disclosure, "cardiac sound" may be, for example, an oscillation waveform (see FIG. 20, etc.) of the chest observed directly using a radar, or chest oscillations. "Heartbeat" is cardiac pulsation itself. The heart rate interval, the number of heart beats, and the like may be calculated from heartbeat behaviors.

The electronic device 1 according to one embodiment includes, as the transmission unit, a transmission DAC 21, a transmission circuit 22, a millimeter-wave transmission circuit 23, and the transmission antenna array 24. The electronic device 1 according to one embodiment includes, as the reception unit, the reception antenna array 31, a mixer 32, a reception circuit 33, and a reception ADC 34. The electronic device 1 according to one embodiment may be configured not to include at least any of the functional units illustrated in FIG. 2 or may include a functional unit(s) other than the functional units illustrated in FIG. 2. The electronic device 1 illustrated in FIG. 2 may be configured using a circuit that has a configuration that is basically the same as or similar to that of an ordinary radar using an electromagnetic wave such as a millimeter-band wave. On the other hand, in the electronic device 1 according to one embodiment, signal processing performed by the signal processing unit 10 may include processing different from that of an ordinary radar of related art.

The signal processing unit 10 of the electronic device 1 according to one embodiment is capable of performing control on the overall operation of the electronic device 1, besides control on each functional unit of the electronic device 1. Among them, the signal processing unit 10 performs various kinds of processing on signals dealt with by the electronic device 1. To provide control and processing capabilities for executing various functions, the signal processing unit 10 may include at least one processor such as, for example, a CPU (Central Processing Unit) or a DSP (Digital Signal Processor). The signal processing unit 10 may be collectively embodied by one processor, may be embodied by some processors, or may be embodied by discrete individual processors. The processor may be embodied as a single integrated circuit. The integrated circuit is also referred to as "IC". The processor may be embodied as a plurality of integrated circuits and discrete circuits connected communicably. The processor may be embodied based on various other known technologies. In one embodiment, the signal processing unit 10 may be configured as, for example, a CPU(s) (hardware) and a program(s) (software) run by the CPU. The signal processing unit 10 may include, as appropriate, a storage unit (memory) necessary for operation of the signal processing unit 10.

The signal generation processing unit 11 of the signal processing unit 10 generates a signal to be transmitted from the electronic device 1. In the electronic device 1 according to one embodiment, the signal generation processing unit 11 may generate a transmission signal such as, for example, a chirp signal (transmission chirp signal). In particular, the signal generation processing unit 11 may generate a signal (linear chirp signal) whose frequency changes linearly in a periodic manner. For example, the signal generation processing unit 11 may generate a chirp signal whose frequency increases linearly in a periodic manner from 77 GHz to 81 GHz as time elapses. For example, the signal generation processing unit 11 may generate a signal whose frequency repeats a linear increase (up-chirp) from 77 GHz to 81 GHz and a decrease (down-chirp) in a periodic manner as time elapses. For example, the signal generated by the signal generation processing unit 11 may be preset at the signal processing unit 10. For example, the signal generated by the signal generation processing unit 11 may be pre-stored in any storage unit or the like of the signal processing unit 10. Since a chirp signal used in a technical field of a radar and the like is known, detailed description thereof will be omitted, or the description thereof will be simplified, where appropriate. The signal generated by the signal generation processing unit 11 is supplied to the transmission DAC 21. Therefore, the signal generation processing unit 11 may be connected to the transmission DAC 21.

The transmission DAC (digital-to-analog converter) 21 has a function of converting a digital signal supplied from the signal generation processing unit 11 into an analog signal. The transmission DAC 21 may include an ordinary digital-to-analog converter. The signal having been converted into an analog format by the transmission DAC 21 is supplied to the transmission circuit 22. Therefore, the transmission DAC 21 may be connected to the transmission circuit 22.

The transmission circuit 22 has a function of converting the signal having been converted into the analog format by the transmission DAC 21 into a signal of an intermediate frequency (IF) band. The transmission circuit 22 may include an ordinary IF-band transmission circuit. The signal having been processed by the transmission circuit 22 is supplied to the millimeter-wave transmission circuit 23. Therefore, the transmission circuit 22 may be connected to the millimeter-wave transmission circuit 23.

The millimeter-wave transmission circuit 23 has a function of transmitting, as a millimeter wave (RF wave), the signal having been processed by the transmission circuit 22. The millimeter-wave transmission circuit 23 may include an ordinary millimeter-wave transmission circuit. The signal having been processed by the millimeter-wave transmission circuit 23 is supplied to the transmission antenna array 24. Therefore, the millimeter-wave transmission circuit 23 may be connected to the transmission antenna array 24. The signal having been processed by the millimeter-wave transmission circuit 23 is supplied to the mixer 32, too. Therefore, the millimeter-wave transmission circuit 23 may be connected to the mixer 32, too.

The transmission antenna array 24 is an array of the plurality of transmission antennas. In FIG. 2, the configuration of the transmission antenna array 24 is simplified. The transmission antenna array 24 transmits the signal having been processed by the millimeter-wave transmission circuit 23 to the outside of the electronic device 1. The transmission antenna array 24 may include a transmission antenna array used in an ordinary millimeter-wave radar.

As described above, the electronic device 1 according to one embodiment includes a transmission antenna (the transmission antenna array 24) and is capable of transmitting a transmission signal (for example, a transmission chirp signal) as a transmission wave from the transmission antenna array 24.

Suppose that, for example, as illustrated in FIG. 2, an object entity such as the person 200 who is the subject is located in the neighborhood of the electronic device 1. In this case, the object entity such as the person 200 who is the subject reflects at least a part of a transmission wave transmitted from the transmission antenna array 24. At least a part of the transmission wave transmitted from the transmission antenna array 24, the part being reflected off the object entity such as the person 200 who is the subject, can come back toward the reception antenna array 31.

The reception antenna array 31 receives the reflected wave. The reflected wave mentioned here may be at least a part of a reflection, of the transmission wave transmitted from the transmission antenna array 24, reflected off the object entity such as the person 200 who is the subject.

The reception antenna array 31 is an array of the plurality of reception antennas. In FIG. 2, the configuration of the reception antenna array 31 is simplified. The reception antenna array 31 receives the reflected wave coming back due to reflection of the transmission wave transmitted from the transmission antenna array 24. The reception antenna array 31 may include a reception antenna array used in an ordinary millimeter-wave radar. The reception antenna array 31 supplies the reception signal received as the reflected wave to the mixer 32. Therefore, the reception antenna array 31 may be connected to the mixer 32.

The mixer 32 performs conversion, into an intermediate frequency (IF) band, of the signal (transmission signal) having been processed by the millimeter-wave transmission circuit 23 and the reception signal having been received by the reception antenna array 31. The mixer 32 may include a mixer used in an ordinary millimeter-wave radar. The mixer 32 supplies a signal generated as a result of mixing to the reception circuit 33. Therefore, the mixer 32 may be connected to the reception circuit 33.

The reception circuit 33 has a function of performing analog processing on the signal having been converted into the IF band by the mixer 32. The reception circuit 33 may include a reception circuit compliant with ordinary IF-band conversion. The signal having been processed by the reception circuit 33 is supplied to the reception ADC 34. Therefore, the reception circuit 33 may be connected to the reception ADC 34.

The reception ADC (analog-to-digital converter) 34 has a function of converting an analog signal supplied from the reception circuit 33 into a digital signal. The reception ADC 34 may include an ordinary analog-to-digital converter. The signal having been digitized by the reception ADC 34 is supplied to the reception signal processing unit 12 of the signal processing unit 10. Therefore, the reception ADC 34 may be connected to the signal processing unit 10.

The reception signal processing unit 12 of the signal processing unit 10 has a function of performing various kinds of processing on the digital signal supplied from the reception DAC 34. For example, based on the digital signal supplied from the reception DAC 34, the reception signal processing unit 12 calculates the distance from the electronic device 1 to the object entity such as the person 200 who is the subject (range measurement). Based on the digital signal supplied from the reception DAC 34, the reception signal processing unit 12 calculates the relative velocity of the object entity such as the person 200 who is the subject in relation to the electronic device 1 (velocity measurement). Based on the digital signal supplied from the reception DAC 34, the reception signal processing unit 12 calculates the angle of azimuth, as viewed from the electronic device 1, of the object entity such as the person 200 who is the subject (angle measurement). Specifically, the reception signal processing unit 12 may receive an input of I/Q-converted data. Upon receiving an input of this kind of data, the reception signal processing unit 12 performs fast Fourier transform in each of a distance (range) direction and a speed (velocity) direction (2D-FFT). After that, the reception signal processing unit 12 performs processing of UART (Universal Asynchronous Receiver Transmitter) and/or CFAR (Constant False Alarm Rate) to remove noise points, thereby suppressing false alarms and making their probabilities constant. Then, the reception signal processing unit 12 performs angle-of-arrival estimation on points satisfying the CFAR criterion to obtain the position of the object entity such as the person 200 who is the subject. Information generated as the results of range measurement, velocity measurement, and angle measurement performed by the reception signal processing unit 12 may be supplied to the heartbeat extraction unit 13.

The heartbeat extraction unit 13 extracts information regarding a heartbeat from the information having been generated by the reception signal processing unit 12. A more detailed description will be given later about operation of extracting the information regarding a heartbeat by the heartbeat extraction unit 13. The information regarding a heartbeat having been extracted by the heartbeat extraction unit 13 may be supplied to the calculation unit 14.

The calculation unit 14 performs various kinds of calculation processing and/or computation processing, etc. on the information supplied from the heartbeat extraction unit 13 regarding a heartbeat. A more detailed description of the various kinds of calculation processing and/or computation processing, etc. performed by the calculation unit 14 will be given later. Various kinds of information having been subjected to the calculation processing and/or the computation processing, etc. by the calculation unit 14 may be supplied to, for example, a communication interface 50 or the like. Therefore, the calculation unit 14 and/or the signal processing unit 10 may be connected to the communication interface 50. The various kinds of information having been subjected to the calculation processing and/or the computation processing, etc. by the calculation unit 14 may be supplied to any functional component other than the communication interface 50.

The communication interface 50 includes an interface configured to output, for example, to an external device 60 or the like, information supplied from the signal processing unit 10. The communication interface 50 may output, to the external device 60 or the like, information on at least one selected from the group consisting of the position, velocity, and angle of the object entity such as the person 200 who is the subject as, for example, a signal of CAN (Controller Area Network) or the like. For example, the information on at least one selected from the group consisting of the position, velocity, and angle of the object entity such as the person 200 who is the subject may be supplied via the communication interface 50 to the external device 60 or the like. Therefore, the communication interface 50 may be connected to the external device 60 or the like.

As illustrated in FIG. 2, the electronic device 1 according to one embodiment may be connected to the external device 60 via the communication interface 50 through wired or wireless connection. In one embodiment, the external device 60 may include any computer and/or any control device, etc.
The electronic device 1 according to one embodiment may include the external device 60. Various kinds of configuration can be adopted for the external device 60, in accordance with how the information on a heartbeat and/or cardiac sound detected by the electronic device 1 is used. Therefore, a more detailed description of the external device 60 is not given here.

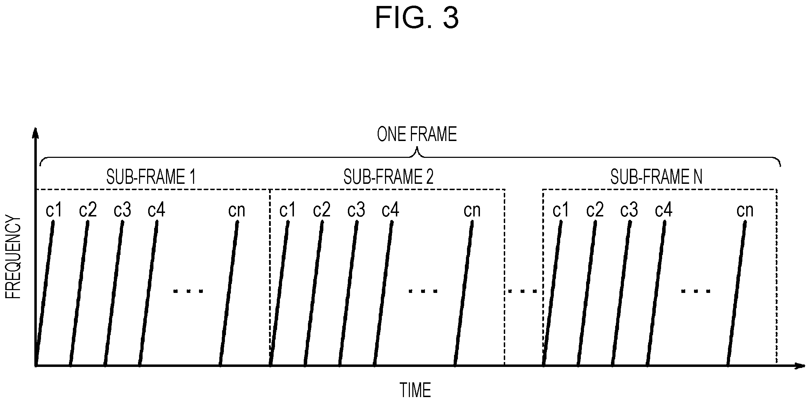

FIG. 3 is a diagram for explaining an example of chirp signals generated by the signal generation processing unit 11 of the signal processing unit 10.

FIG. 3 illustrates a structure of one frame with respect to time when an FCM (Fast-Chirp Modulation) scheme is used. FIG. 3 illustrates an example of a reception signal conforming to the FCM scheme. The FCM is a scheme in which chirp signals denoted by c1, c2, c3, c4, ..., cn in FIG. 3 are repeated at short intervals (equal to or greater than a round-trip time of an electromagnetic wave between the radar and the radar target, calculated from, for example, the maximum ranging distance). In the FCM, for convenience of signal processing of a reception signal, transmission/reception processing is often performed with division in unit of sub-frames such as those illustrated in FIG. 3.

In FIG. 3, the horizontal axis represents elapsed time and the vertical axis represents frequency. In the example illustrated in FIG. 3, the signal generation processing unit 11 generates linear chirp signals whose frequency changes linearly in a periodic manner. In FIG. 3, the individual chirp signals are denoted by c1, c2, c3, c4, ..., cn. As illustrated in FIG. 3, the frequency of each chirp signal increases linearly as time elapses.

In the example illustrated in FIG. 3, one sub-frame includes several chirp signals c1, c2, c3, c4, ..., cn. That is, each of sub-frames such as a sub-frame 1 and a sub-frame 2 illustrated in FIG. 3 includes several chirp signals c1, c2, c3, c4, ..., cn. In the example illustrated in FIG. 3, one frame (each single frame) includes several sub-frames such as the sub-frames 1, 2, ..., N. That is, one frame illustrated in FIG. 3 includes N sub-frames. The one frame illustrated in FIG. 3 may serve as a frame 1, which may be followed by a frame 2, a frame 3, and so on. Similarly to the frame 1, each of these frames may include N sub-frames. A frame interval of a predetermined length may be included between frames. One frame illustrated in FIG. 3 may have a length of approximately 30 ms to 50 ms, for example.

In the electronic device 1 according to one embodiment, the signal generation processing unit 11 may generate a transmission signal as any number of frames. In FIG. 3, illustration of a part of chirp signals is omitted. As described above, a relationship between time and frequency of a transmission signal generated by the signal generation processing unit 11 may be stored in, for example, the storage unit or the like of the signal processing unit 10.

As described above, the electronic device 1 according to one embodiment may transmit a transmission signal constituted by sub-frames each including a plurality of chirp signals. The electronic device 1 according to one embodiment may transmit a transmission signal constituted by frames each including a predetermined number of sub-frames.

In the description given below, the electronic device 1 is assumed to transmit a transmission signal having a frame structure illustrated in FIG. 3. However, the frame structure illustrated in FIG. 3 is just an example. For example, any number of chirp signals may be included in one sub-frame. That is, in one embodiment, the signal generation processing unit 11 may generate a sub-frame including any number (for example, any plural number) of chirp signals. The sub-frame structure illustrated in FIG. 3 is also just an example. For example, any number of sub-frames may be included in one frame. That is, in one embodiment, the signal generation processing unit 11 may generate a frame including any number (for example, any plural number) of sub-frames. The signal generation processing unit 11 may generate signals having different frequencies. The signal generation processing unit 11 may generate a plurality of discrete signals having bandwidths in which frequencies f are different from each other.

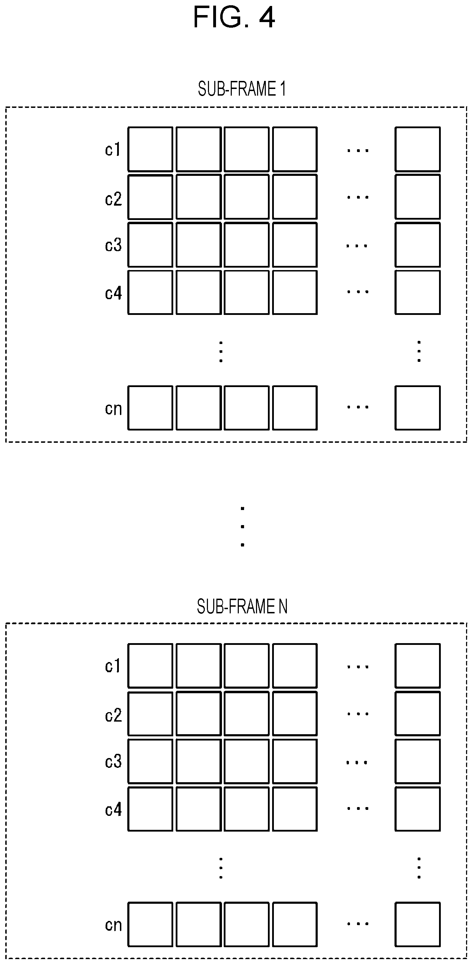

FIG. 4 is a diagram illustrating a part of the sub-frames of FIG. 3 in a different manner. FIG. 4 illustrates each sample of a reception signal obtained as a result of performing two-dimensional fast Fourier transform (2D-FFT), which is processing performed at the reception signal processing unit 12 of the signal processing unit 10 (FIG. 2) when the transmission signal illustrated in FIG. 3 is received.

As illustrated in FIG. 4, the chirp signals c1, c2, c3, c4, ..., cn are stored in each sub-frame such as the sub-frame 1, ..., sub-frame N. In FIG. 4, each of the chirp signals c1, c2, c3, c4, ..., cn consists of samples represented by a row of squares arranged in a horizontal direction. The reception signal illustrated in FIG. 4 is subjected to 2D-FFT, CFAR, and/or integrated signal processing for each sub-frame, etc. by the reception signal processing unit 12 illustrated in FIG. 2.

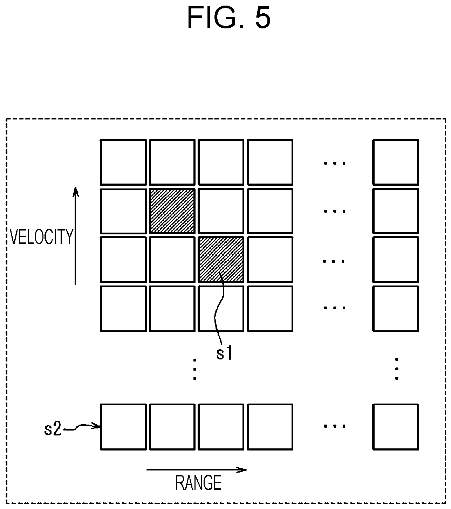

Fig. 5 is a diagram illustrating an example in which a point group on a range-Doppler (distance-velocity) plane is calculated as a result of performing 2D-FFT, CFAR, and integrated signal processing of the sub-frames at the reception signal processing unit 12 illustrated in Fig. 2.

In Fig. 5, the horizontal direction represents range (distance) and the vertical direction represents velocity. A shaded square s1 illustrated in FIG. 5 denotes a point group representing a signal exceeding the threshold of CFAR. An unshaded square s2 illustrated in FIG. 5 denotes a bin (2D-FFT sample) without a point group, not exceeding the threshold of CFAR. For a point group on the range-Doppler plane calculated in FIG. 5, the direction from the radar is calculated by performing direction estimation, and the position and velocity on a two-dimensional plane are calculated as a point group representing the object entity such as the person 200 who is the subject. The direction estimation mentioned here may be calculated using a beam former and/or a subspace method. Typical algorithms for the subspace method are: MUSIC (multiple signal classification), ESPRIT (estimation of signal parameters via rotation invariance technique), and the like.

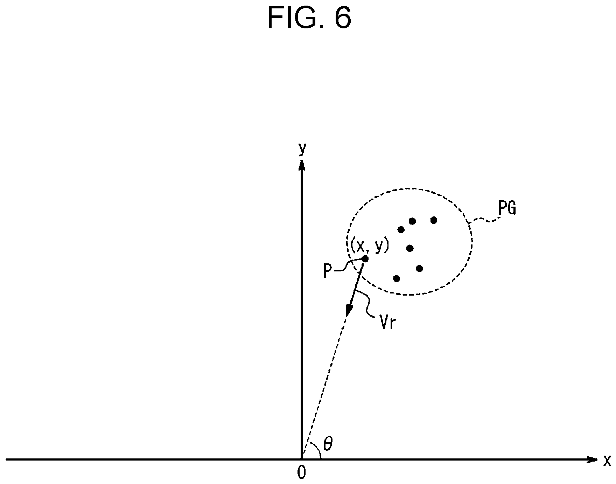

FIG. 6 illustrates an example of results obtained by transforming point group coordinates from the range-Doppler plane illustrated in FIG. 5 to the XY plane after performing the direction estimation by the reception signal processing unit 12. As illustrated in FIG. 6, the reception signal processing unit 12 is capable of plotting a point group PG on the XY plane. The point group PG is made up of individual points P. Each point P has an angle $\theta$ and a velocity Vr in a radial direction in polar coordinates.

Based on at least the results of 2D-FFT and angle estimation, the reception signal processing unit 12 detects an object entity located in a range in which a transmission wave T is transmitted. The reception signal processing unit 12 may perform object detection by performing, for example, clustering processing on the basis of each estimated range information, velocity information, and angle information. A known example of algorithms used for data clustering is DBSCAN (density-based spatial clustering of applications with noise). This is an algorithm for performing density-based clustering. In the clustering processing, for example, average power of points constituting the object entity to be detected may be calculated. The range information, the velocity information, the angle information, and the power information regarding the object entity detected by the reception signal processing unit 12 may be supplied to, for example, the external device 60, etc. via the communication interface 50.

As described above, the electronic device 1 may include a transmission antenna(s) (the transmission antenna array 24), a reception antenna(s) (the reception antenna array 31), and the signal processing unit 10. The transmission antenna array 24 transmits a transmission wave T. The reception antenna array 31 receives a reflected wave R that is the transmission wave T having been reflected. Then, based on a transmission signal transmitted as the transmission wave T and a reception signal received as the reflected wave R, the signal processing unit 10 detects an object entity (for example, the object entity such as the person 200 who is the subject) that reflects the transmission wave T.

A further explanation will now be given of estimating the direction of an arrival wave by the antenna array of the electronic device 1 according to one embodiment.

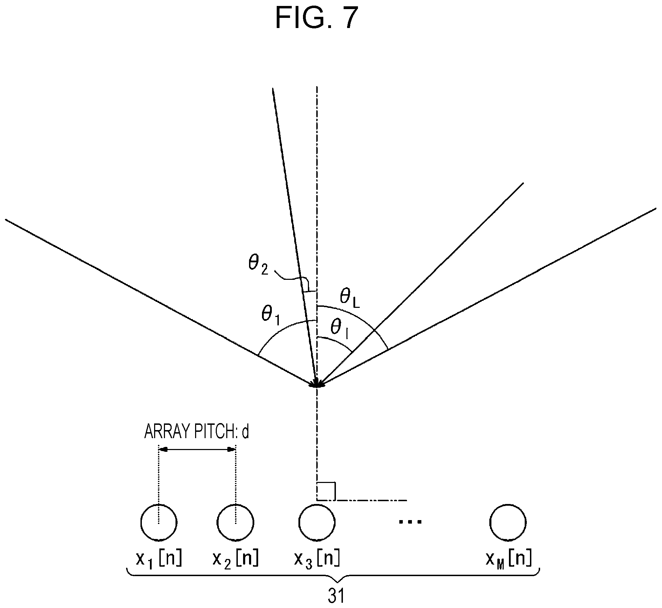

FIG. 7 is a diagram for explaining the configuration of the reception antenna array 31 of the electronic device 1 according to one embodiment, and explaining the principle of estimating the direction of an arrival wave by the reception antenna array 31. FIG. 7 illustrates an example of receiving a radio wave by the reception antenna array 31.

As illustrated in FIG. 7, the reception antenna array 31 may be an array of sensors such as reception antennas arranged in a straight line. As illustrated in FIG. 7, in one embodiment, the reception antenna array 31 may include a plurality of reception antennas arranged in a straight line. In FIG. 7, small circles represent a plurality of antennas such as antennas $x_1$, $x_2$, $x_3$, ..., $x_M$ of the reception antenna array 31. The reception antenna array 31 may include any plurality of antennas. As illustrated in FIG. 7, the plurality of antennas constituting the reception antenna array 31 are arranged at intervals of an array pitch d. A sensor array in which sensors (antennas, ultrasonic transducers, microphones, or the like) corresponding to various physical waves are arranged to form an array as described here is referred to also as a uniform linear array (ULA). As illustrated in FIG. 7, the physical waves (such as electromagnetic waves or sound waves) arrive from various directions such as, for example, $\theta_1$ and $\theta_2$. The directions $\theta_1$ and $\theta_2$ mentioned here may be the angles of arrival described above. The sensor array such as the reception antenna array 31 is capable of estimating the direction of arrival (angle of arrival) by using a phase difference caused between measured values of the sensors in accordance with the directions of arrival of the physical waves. The method of estimating the direction of arrival of a wave as described here is referred to also as angle-of-arrival estimation or direction-of-arrival estimation (DoA).

In the electronic device 1 according to one embodiment, at least one of the transmission antenna array 24 or the reception antenna array 31 may be a linear array of a plurality of antennas. This enables directivity to be narrowed appropriately when transmitting/receiving a radio wave at, for example, a millimeter-wave radar. To transmit a transmission wave, a beam former is often used for controlling the direction of a transmission beam. On the other hand, to receive a reflected wave, a subspace method (the above-mentioned MUSIC, ESPRIT, etc.) is often used, rather than a beam former, for estimating the direction of arrival of the reflected wave. In beamforming and sub-spacing, with regard to electromagnetic waves coming from various directions, a phase difference arises between measured values of the sensors of the ULA illustrated in FIG. 7 in accordance with the directions of arrival. Therefore, utilizing the phase difference enables estimating the direction of arrival of the reflected wave.

Next, a further explanation will now be given of angle estimation in two directions of an arrival wave by the antenna array of the electronic device 1 according to one embodiment.

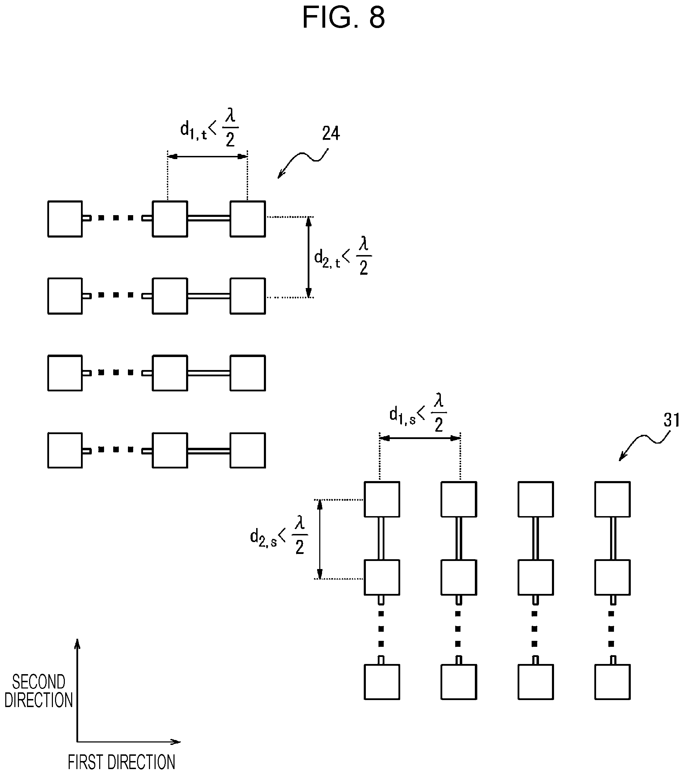

FIG. 8 is a diagram illustrating an example of antenna arrangement for direction-of-arrival estimation for two orthogonal angles.

As illustrated in FIG. 8, in the electronic device 1 according to one embodiment, the transmission antenna array 24 and/or the reception antenna array 31 may include an array of a plurality of patch antenna units.

In the transmission antenna array 24 illustrated in FIG. 8, one patch antenna unit may include a plurality of elements connected electrically in a first direction illustrated therein. In each patch antenna unit, the plurality of elements may be connected electrically via wiring such as, for example, a strip line on a substrate. In each patch antenna unit, the plurality of elements may be spaced apart from one another by an interval $d_{1,t}$, which is shorter than a half of a wavelength $\lambda$ of a transmission wave. In FIG. 8, any number, two or more, of elements may be electrically connected to configure each patch antenna unit.

As illustrated in FIG. 8, the transmission antenna array 24 may be an array of the plurality of patch antenna units arranged in a second direction illustrated therein. The patch antenna units may be spaced apart from one another by an interval $d_{2,t}$, which is shorter than a half of the wavelength $\lambda$ of the transmission wave. In one embodiment, the transmission antenna array 24 may include any number, two or more, of patch antenna units.

As illustrated in FIG. 8, in one embodiment, the reception antenna array 31 may have a layout in which the arrangement of the plurality of elements in the transmission antenna array 24 is altered. That is, in the reception antenna array 31 illustrated in FIG. 8, one patch antenna unit may include a plurality of elements connected electrically in the second direction illustrated therein. In each patch antenna unit, the plurality of elements may be connected electrically via wiring such as, for example, a strip line on the substrate. In each patch antenna unit, the plurality of elements may be spaced apart from one another by an interval $d_{2,s}$, which is shorter than a half of the wavelength $\lambda$ of the transmission wave. In FIG. 8, any number, two or more, of elements may be electrically connected to configure each patch antenna unit.

As illustrated in FIG. 8, the reception antenna array 31 may be an array of the plurality of patch antenna units arranged in the first direction illustrated therein. The patch antenna units may be spaced apart from one another by an interval $d_{1,s}$, which is shorter than a half of the wavelength $\lambda$ of the transmission wave. In one embodiment, the reception antenna array 31 may include any number, two or more, of patch antenna units.

All of the elements included in the transmission antenna array 24 and the reception antenna array 31 may be disposed on the same plane (for example, on a surface layer of the same substrate). The transmission antenna array 24 and the reception antenna array 31 may be disposed in proximity to each other (monostatic). The first direction and the second direction illustrated in FIG. 8 may be geometrically orthogonal to each other.

The transmission antenna array 24 and the reception antenna array 31 illustrated in FIG. 8 make it possible to narrow the respective directivities of the transmission antennas and the reception antennas appropriately. A beam former in the second direction illustrated in FIG. 8 can be realized by using the transmission antenna array 24 illustrated in FIG. 8 to control the direction of transmitting each transmission wave at each timing of transmitting the transmission wave (transmission signal). Using the reception antenna array 31 illustrated in FIG. 8 realizes estimation of the direction of arrival of a reflected wave regarding the first direction illustrated in FIG. 8. In this way, the direction of arrival of the reflected wave can be estimated regarding the two angles that are substantially orthogonal to each other. Therefore, a point group indicating the object entity such as the person 200 who is the subject can be acquired in a three-dimensional manner.

Next, a method as to how the electronic device 1 according to one embodiment detects a heartbeat of the person 200 who is the subject will now be described.

The electronic device 1 according to one embodiment transmits a transmission wave such as a millimeter-wave radar toward the person 200 who is the subject, and measures (estimates) the heartbeat of the person 200 who is the subject on the basis of a result of receiving a wave reflected off the chest where the heart of the person 200 who is the subject exists. As described earlier, the subject 200 may be a human or an animal. In this case, components expected to draw an envelope of a heartbeat can be extracted by, for example, performing frequency filtering on oscillations at the position where the presence of the person 200 who is the subject is detected by means of a radar. Then, upon extracting the components expected to draw an envelope of a heartbeat, an interval between a peak and another peak of the envelope is defined as a heart rate interval; this enables calculation of an approximate value of the heart rate interval. Approximation of a peak on an envelope of a heartbeat to an R peak in an electrocardiogram can be employed here. Therefore, similarly to the terms used in electrocardiography and the like, the "heart rate interval" is referred to also as an RR interval or RRI.

Studied below is a method of estimating the heart rate interval of the person 200 who is the subject on the basis of the results of performing 2D-FFT, CFAR processing, and direction-of-arrival estimation described above, etc. First, how a heartbeat or a body motion of a person is expressed as the result of 2D-FFT performed in FIGs. 4 and 5 will now be described.

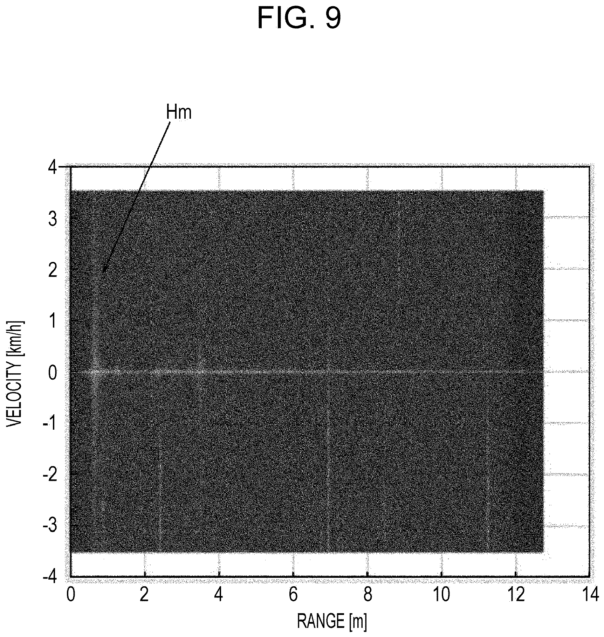

FIG. 9 is a diagram illustrating an example of the result of 2D-FFT performed by receiving a reflected wave of a transmission wave transmitted toward the person 200 who is the subject. FIG. 9 illustrates, as the result of 2D-FFT, a spectrum representing cardiac sound and a body motion of the person 200 who is the subject. In FIG. 9, the horizontal axis represents range and the vertical axis represents velocity. The signal processing unit 10 (for example, the heartbeat extraction unit 13) of the electronic device 1 according to one embodiment may, for example, extract a peak Hm illustrated in FIG. 9 as a body motion such as a heartbeat of the person 200 who is the subject. The spectral component indicated by the peak Hm illustrated in FIG. 9 includes not only cardiac sound of the person 200 who is the subject and the envelope of the cardiac sound, but also a body motion. To extract a heart rate interval, extraction of a body motion or the like is needed. Therefore, for example, performing frequency filtering is supposed. As the frequency filtering performed here, for example, applying a band pass filter directed to 0.5 Hz or higher and 10 Hz or lower, a high pass filter, and/or a low pass filter, etc. is supposed.

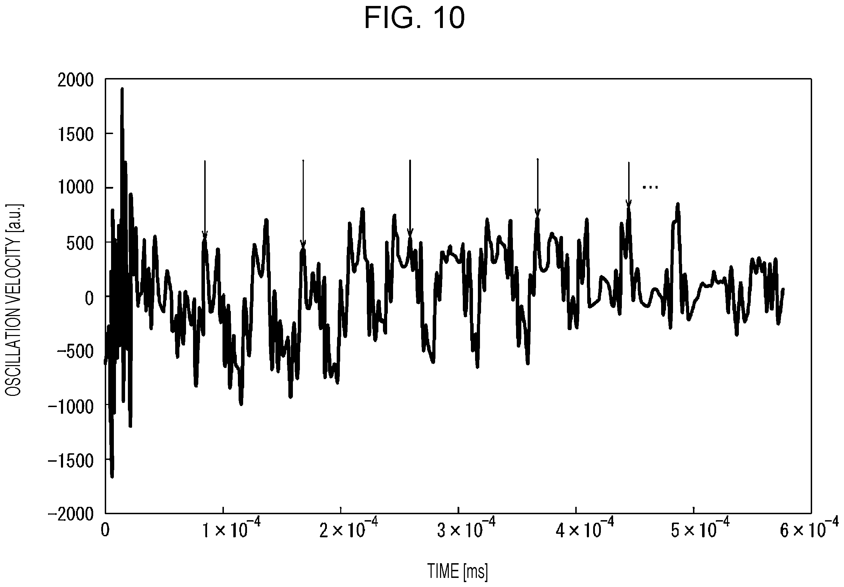

FIG. 10 is a diagram for explaining a method of detecting peaks on the basis of an envelope waveform of cardiac sound obtained through the frequency filtering described above. The graph illustrated in FIG. 10 gives an example of changes over time in the envelope waveform of cardiac sound obtained through the frequency filtering described above. The envelope waveform of FIG. 10 includes many peaks as illustrated therein. On the other hand, the beating of the person 200 who is the subject is known to fall within a range of approximately 50 to 130 beats per minute. Therefore, an approximate cardiac sound interval of the person 200 who is the subject can be calculated by selecting, from among many peaks illustrated in FIG. 10, peaks the time interval between which is within a range from 0.4 second to 0.8 second, which is the reciprocal of the number of beats per minute. For example, peaks indicated by down-pointing arrows in FIG. 10 may be selected as the approximate cardiac sound interval of the person 200 who is the subject.

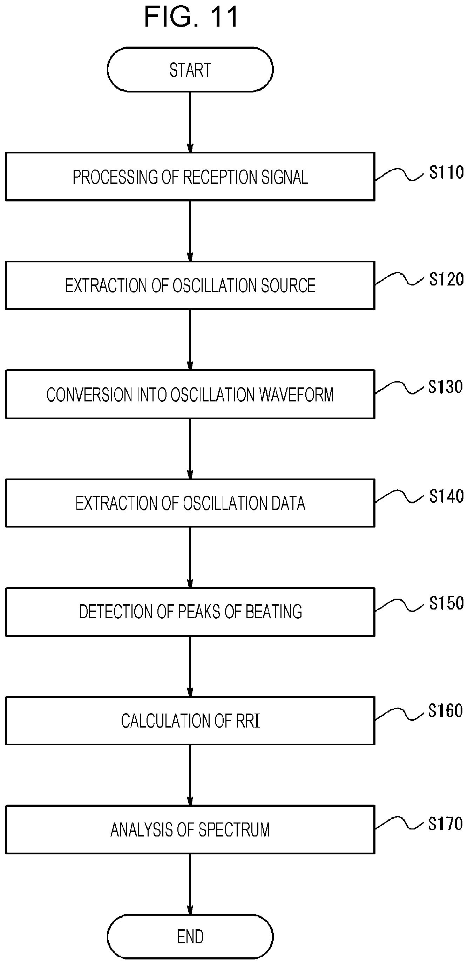

FIG. 11 is a flowchart illustrating an example of operation for estimating the heart rate interval described above. Described below while referring to FIG. 11 is an overview of the operation for estimating the heart rate interval described above.

FIG. 11 illustrates the operation performed after receiving the reflected wave by the electronic device 1 according to one embodiment. That is, as the premise on which the operation illustrated in FIG. 11 is based, the electronic device 1 illustrated in FIG. 2 transmits a transmission wave (transmission signal) from the transmission antenna array 24. Then, at least a part of the transmission wave transmitted from the electronic device 1 turns into a reflected wave due to reflection off (the chest of) the person 200 who is the subject. Then, the electronic device 1 illustrated in FIG. 2 receives such a reflected wave via the reception antenna array 31. The operation illustrated in FIG. 11 thereafter starts.

Upon the start of the operation illustrated in FIG. 11, first, in step S110, the signal processing unit 10 of the electronic device 1 processes the received signal (reception signal). The signal processing performed in step S110 may include, for example, 2D-FFT, CFAR processing, and/or direction-of-arrival estimation described above. This operation may be performed by, for example, the reception signal processing unit 12 of the signal processing unit 10.

Next, in step S120, the signal processing unit 10 extracts the source of oscillations from the information having been processed in step S110. The signal processing performed in step S120 may include, for example, processing of performing filtering on data obtained as the result of performing 2D-FFT. In step S120, the signal processing unit 10 may extract spectral components at only the position of presence of the person 200 who is the subject. Various known methods may be used for pinpointing the position of presence of the person 200 who is the subject. This operation may be performed by, for example, the heartbeat extraction unit 13 of the signal processing unit 10.

Next, in step S130, the signal processing unit 10 converts the results of processing having been performed upstream into an oscillation waveform. The processing performed in step S130 may include, for example, processing of extracting phase information from IQ data. In step S130, the signal processing unit 10 may extract oscillation data including cardiac sound from the spectral components of 2D-FFT processing of the person 200 who is the subject, which have been extracted in step S120. This operation may be performed by, for example, the heartbeat extraction unit 13 of the signal processing unit 10.

Next, in step S140, the signal processing unit 10 extracts oscillation data from the results of processing having been performed upstream. The processing performed in step S140 may include, for example, frequency filtering. In step S140, by performing frequency filtering, the signal processing unit 10 may extract a low frequency signal including the envelope of cardiac sound of the person 200 who is the subject. This operation may be performed by, for example, the heartbeat extraction unit 13 of the signal processing unit 10.

Next, in step S150, the signal processing unit 10 extracts peaks of the beating of the person 200 who is the subject from the results of processing having been performed upstream. In step S150, the signal processing unit 10 may detect peaks of the low frequency signal including the envelope of cardiac sound of the person 200 who is the subject. This operation may be performed by, for example, the calculation unit 14 of the signal processing unit 10.

Next, in step S160, the signal processing unit 10 calculates the RR interval (RRI) of the person 200 who is the subject from the results of processing having been performed upstream. In step S160, the signal processing unit 10 may calculate each peak-to-peak pitch (interval). In step S160, the signal processing unit 10 may calculate the RRI by extracting the interval between the values of the peaks having been detected in step S150. This operation may be performed by, for example, the calculation unit 14 of the signal processing unit 10.

Next, in step S170, the signal processing unit 10 performs spectrum analysis on the results of processing having been performed upstream. In step S 170, the signal processing unit 10 may calculate the heart rate variability (HRV) of the person 200 who is the subject by calculating the spectral density of time-series data of the RRI. For calculating the power spectral density of the time-series data of the RRI, for example, Welch's method or the like may be used. This operation may be performed by, for example, the calculation unit 14 of the signal processing unit 10.

As described above, by performing frequency filtering on oscillations at the position where the person 200 who is the subject is present, the electronic device 1 according to one embodiment extracts components expected to draw an envelope of a heartbeat, and calculate an interval between peaks of the envelope as a heart rate interval. In this way, the electronic device 1 according to one embodiment is capable of calculating an approximate heart rate interval of the person 200 who is the subject.

In calculating the heart rate interval described above, there exist some peaks during a period of a few tens of milliseconds, for example, as illustrated in FIG. 10. Therefore, selecting peaks of a heartbeat involves a certain degree of uncertainty. For this reason, an error of a few tens of millisecond or so is inherent in accuracy of the calculated heart rate interval. For example, in calculating the heart rate interval described above, at least an error of 20 ms or so occurs, as compared with an instantaneous RRI acquired by means of an electrocardiographic monitor. With this level of accuracy, it is difficult to calculate the heart rate variability (HRV) to conduct an autonomic-nerve and/or emotion analysis of a person. That is, in calculating the heart rate interval described above, it is difficult to conduct a more sophisticated medical analysis by extracting cardiac sound.

In view of the above, for example, it is conceivable to further cut high-frequency-side peaks by performing frequency filtering to make the time interval of the heartbeat fall within a range from 0.4 second to 0.8 second. However, reducing an error of a few tens of millisecond or so seems to be difficult because this does not exceed the accuracy of information on the signal illustrated in FIG. 10.

In calculating the heart rate interval described above, it is not cardiac sound that is extracted. Therefore, if the method described above is used, it also seems to be difficult to obtain information that will contribute to making a diagnosis (for example, auscultation in medical examination or the like) utilizing acoustic properties of cardiac sound itself.

For a solution, the electronic device 1 according to one embodiment features a further improvement on the method described above. Featuring this improvement, the electronic device 1 according to one embodiment analyzes cardiac sound itself to extract an accurate heart rate interval, for example, by extracting the cardiac sound by means of a radar using a high frequency band of a millimeter wave or higher. This method will be described below.

In order to extract cardiac sound of the person 200 who is the subject with high accuracy, the electronic device 1 according to one embodiment performs appropriate signal processing in an appropriate sequential order on a signal (chirp signal) that the electronic device 1 receives, thereby narrowing down a subspace where signal components of cardiac sound of the person 200 who is the subject exists, and a subspace basis. The electronic device 1 according to one embodiment may, in a linear space where a signal of cardiac sound of the person 200 who is the subject exists, adopt different basis vectors, and may extract a subspace based on respective coordinates while describing a space using an appropriate coordinate system. Through this processing, the electronic device 1 according to one embodiment is capable of searching for a subspace where cardiac sound of the person 200 who is the subject exists. In one embodiment, for such a coordinate system, one suited to the purpose may be used. For example, a coordinate system of a space having been subjected to 2D-FFT processing, a coordinate system of a time-series signal taken through contraction of a chirp signal timewise, a coordinate system based on a Fourier transform thereof, or a coordinate system based on continuous/discrete wavelet, etc. may be used.

The electronic device 1 according to one embodiment may perform the following characteristic processing on a received signal through step-by-step procedures. The characteristic processing performed by the electronic device 1 according to one embodiment will be described schematically in order below.

First Step: Reduction in Dimensions

In a first step, an appropriate window function is applied to 2D-FFT processing of a received chirp signal, and, in addition, micro-Doppler components only of a point group of the presence of the person 200 who is the subject are extracted through direction-of-arrival estimation.

Second Step: Reduction in Dimensions of Subspace

In a second step, principal component analysis and/or singular value decomposition, etc. of a set of time-series waveforms of oscillations having been extracted in the first step is/are performed on the micro-Doppler signal.

Third Step: Reduction in Dimensions of Subspace

In a third step, frequency filtering is performed by using at least one selected from the group consisting of short-time Fourier transform, continuous wavelet transform, and bandpass filtering.

Fourth Step: Reduction in Dimensions of Subspace

In a fourth step, cardiac sound is extracted by performing multi-resolution analysis based on discrete wavelet transform by applying an appropriate wavelet function and an appropriate scaling function to the cardiac sound.

With the electronic device 1 according to one embodiment, by acquiring an envelope waveform of cardiac sound obtained through the processing in each of the above-described steps, its positive/negative peaks can be detected, and the RRI can be calculated with high accuracy by appropriately calculating the interval between the peaks. As the method for acquiring the envelope waveform, for example, envelope waveform calculation based on continuous wavelet transform or Hilbert transform, etc. may be used.

Next, operation of the electronic device 1 according to one embodiment will now be described in more detail.

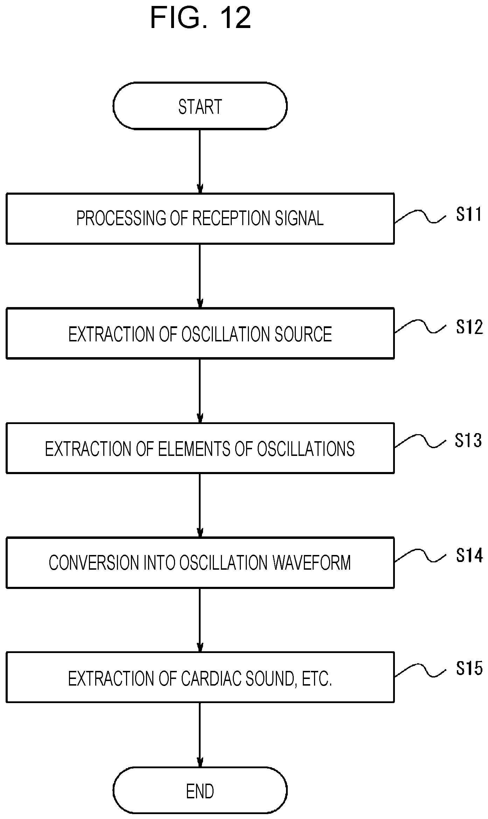

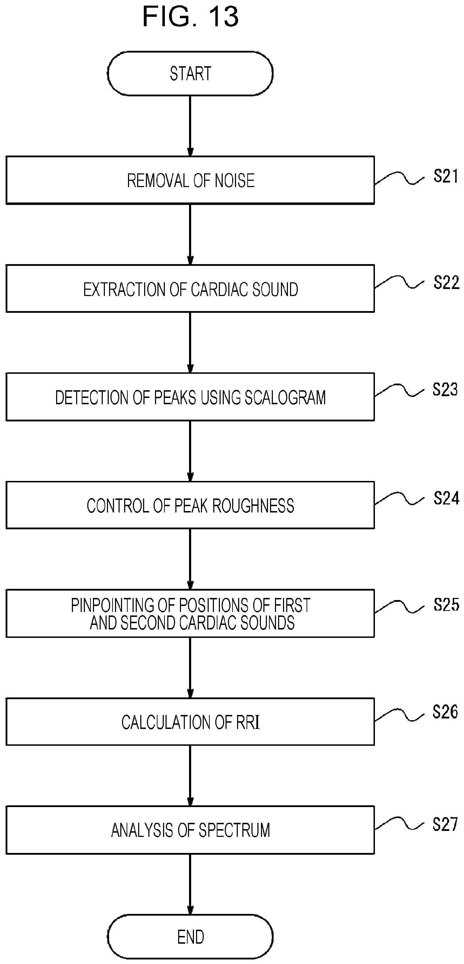

FIG. 12 is a flowchart illustrating an example of operation performed by the electronic device 1 according to one embodiment. FIG. 13 is a flowchart illustrating an example of operation in step S15 of FIG. 12 in more detail. A flow of operation performed by the electronic device 1 according to one embodiment will be described schematically below while referring to FIGs. 12 and 13.

Step S11 of FIG. 12 can be executed in the same manner as in the operation in step S 110 illustrated in FIG. 11, or in a similar manner. That is, upon the start of the operation illustrated in FIG. 12, first, in step S11, the signal processing unit 10 of the electronic device 1 processes the received signal (reception signal). The signal processing performed in step S11 may include, for example, 2D-FFT, CFAR processing, and/or direction-of-arrival estimation described above. This operation may be performed by, for example, the reception signal processing unit 12 of the signal processing unit 10.

Next, in step S12, the signal processing unit 10 extracts the source of oscillations from the information having been processed in step S11. However, the processing of extracting the source of oscillations in step S12 may be different from the processing of extracting the source of oscillations in step S120 illustrated in FIG. 11. In step S12, the signal processing unit 10 may extract only the region of presence of the person 200 who is the subject, by means of an appropriate window function, on the range-Doppler plane having been calculated through 2D-FFT. In step S12, the signal processing unit 10 may use, as the window function, for example, a hanning window, a hamming window, a Blackman-Harris window, or the like.

In step S12, the signal processing unit 10 may perform extraction of the source of oscillations including a body motion including cardiac sound and/or respiration of the person 200 who is the subject on the basis of, for example, the following steps.

(First Step)

Based on the results of direction-of-arrival estimation (see FIGs. 5 and 6), the signal processing unit 10 classifies a point group exceeding the threshold of CFAR on the range-Doppler plane into areas of angles having been determined in advance. For example, classification into areas A to C of the following angles may be performed, where $\theta$ denotes an angle on an xy plane illustrated in FIG. 6:

Area A: -10 deg. $< \theta <$ 10 deg.
Area B: -20 deg. $< \theta \leq$ -10 deg.
Area C: 10 deg. $\leq \theta <$ 20 deg.

(Second Step)

The signal processing unit 10 applies clustering to, among those in a certain angular area group into which the classification has been performed in the first step, the point group exceeding the threshold of CFAR on the range-Doppler plane, as labeled as s1 in FIG. 5. For example, a DBSCAN method or the like may be used for the clustering performed here.

(Third Step)

Assuming that L clusters have been processed in the second step, the signal processing unit 10 compares, for the l-th cluster, a deviation $D_{dev}[l]$ in the number of bins in the Doppler direction with a certain formula value $D_{dev, th}$. As the result of this comparison, the signal processing unit 10 determines only those for which $D_{dev}[l] \geq D_{dev, th}$ holds as the person 200 who is the subject, and sets flag HF[l] ON for them. That is, for example, the signal processing unit 10 may perform the following processing using a pseudo code:

$$\text{for } l = 1 \text{ to } L \text{ do}$$
$$\text{if } D_{dev}[l] \geq D_{dev,th}$$
$$HF[l] = 1$$
$$\text{else}$$
$$HF[l] = 0$$
$$\text{end if}$$
$$\text{end do}$$

The signal processing unit 10 may perform subsequent processing for only regions of clusters for which the flag HF[l] of the person 200 who is the subject indicates 1. The signal processing unit 10 applies, to the cluster of the number l for which HF[l] = 1 holds, a window function whose center lies on the center bin in the range direction thereof. By this means, the signal processing unit 10 is capable of extracting oscillations including cardiac sound, respiration, and/or a body motion of the person 200 who is the subject. The operation described above may be performed by, for example, the reception signal processing unit 12 or the heartbeat extraction unit 13 of the signal processing unit 10.

Next, in step S13, the signal processing unit 10 extracts elements of oscillations from information on the source of oscillations having been extracted in step S12. In step S13, the signal processing unit 10 performs singular value decomposition (SVD) for removing signal noise in a pre-processed manner. In step S13, the signal processing unit 10 may remove noise whose energy is low. In step S13, the signal processing unit 10 may remove oscillation data having crept in due to Fourier transform uncertainty (i.e., oscillation data except those lying at desired positions illustrated in FIG. 9). The operation described above may be performed by, for example, the reception signal processing unit 12 or the heartbeat extraction unit 13 of the signal processing unit 10.

The next step, S14, can be executed in the same manner as in the operation in step S130 illustrated in FIG. 11, or in a similar manner. That is, in step S14, the signal processing unit 10 converts the results of processing having been performed upstream into an oscillation waveform. The processing performed in step S14 may include, for example, processing of extracting phase information from IQ data. This operation may be performed by, for example, the reception signal processing unit 12 or the heartbeat extraction unit 13 of the signal processing unit 10.

The processing performed in step S13 and step S14 will now be further described.

Let $S_{vib}$ be a received chirp signal set having been extracted on the 2D-FFT plane. A result expressed by Formula (1) shown below can be obtained by performing singular value decomposition of $S_{vib}$.
[Formula 1]

$$S_{vib} = U \ \Sigma \ V^* \in \mathbb{C}^{N \times M} \qquad (1)$$

In Formula (1), U denotes a matrix of left-singular vectors, $\Sigma$ denotes a matrix in which singular values are arranged diagonally, and V denotes a matrix in which right-singular vectors are arranged. In Formula (1), * denotes Hermitian transpose. N denotes the number of samples in one chirp signal, and M denotes the number of chirp snapshots.

Next, the number of row vectors of rows of the left-singular vectors in Formula (1) described above is limited ($U_{ext}$), and the number of diagonal elements in the diagonal matrix of singular values is limited ($\Sigma_{ext}$). By this means, unwanted noise signal components and oscillation components except those lying at desired positions are removed. Consequently, a signal $S_{ext}$ obtained by projecting a desired signal of $S_{vib}$ into a subspace can be expressed by Formula (2) shown below:
[Formula 2]

$$S_{ext} = U_{ext} \ \Sigma_{ext} \ V^* \in \mathbb{C}^{N \times M} \qquad (2)$$

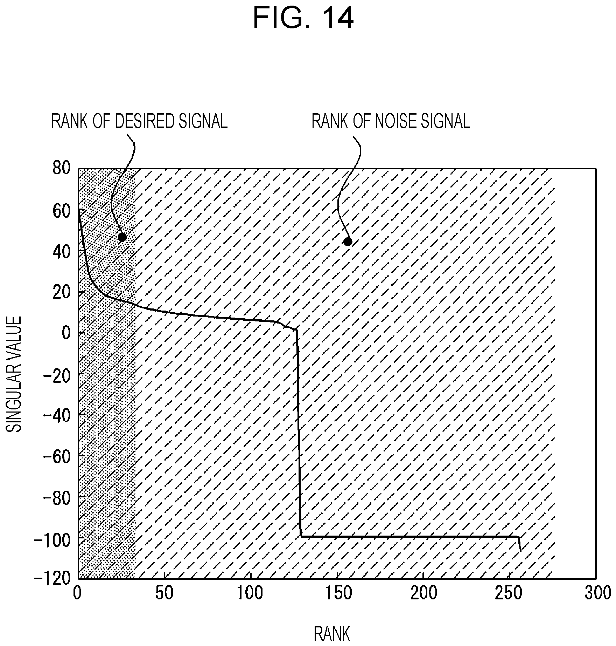

FIG. 14 is a diagram illustrating an example of a relationship between a rank of a desired signal and a rank of a noise signal in singular value decomposition. A signal $p_{ext}$ expressed by Formula (3) shown below is generated by finding a sum total based on the total number N of samples, or a part of the samples, in one chirp signal of $S_{ext}$. The signal $p_{ext}$ represents micro-Doppler. In FIG. 14, the results of SVD are arranged in a descending order of singular values (corresponding to energy), with the horizontal axis representing ranks and the vertical axis representing the singular values. In the present disclosure, a concept that a signal of a certain singular value or greater is always a desired signal is adopted. In this case, a singular-value-rank boundary defining whether a certain signal is a desired signal or not lies at a border portion of a rank region up to 30 inclusive, which is indicated by a dark gray section in the graph of FIG. 14. As illustrated in FIG. 14, the section corresponding to the rank region up to 30 inclusive is the region of the desired signal. In FIG. 14, the desired-signal rank section lies from 1 inclusive to 30 inclusive. In the present disclosure, therefore, it follows that singular vectors corresponding to left/right singular values of the rank region from 1 inclusive to 30 inclusive are taken out.

In the example described above, the desired-signal rank section lies from 1 inclusive to 30 inclusive. Basically, the maximum rank value may be determined empirically. The maximum rank value (in the present disclosure, the rank 30) may be determined using a statistical method. In the graph of FIG. 14, the singular value takes a nosedive at the rank 130, and signals beyond this rank are basically defined as noise. Therefore, the signals beyond this rank are not needed.

In the graph of FIG. 14, the left/right-singular vectors corresponding to the singular values in the area of the rank 31 to 130 (vectors forming a space of a signal) slightly contain some desired signal components, too. The principal causes of these left/right-singular vectors seem to be unnecessary micro oscillations, and/or artificial noises (artifacts) produced by signal processing of the radar, etc. Therefore, these elements may be disregarded.

[Formula 3]

$$\boldsymbol{p}_{ext} = \textstyle\sum_{n_1 \le n \le n_2} S_{ext}[n, m] \in \mathbb{C}^M \qquad (\,3\,)$$

Oscillation displacement $d_{ext}$ in phase of a complex signal (I/Q signal) $P_{ext}$ can be expressed by Formula (4) as follows:

[Formula 4]

$$\boldsymbol{d}_{ext} = \arg \boldsymbol{p}_{ext} \in \mathbb{C}^M \qquad (\,4\,)$$

By finding a time derivative of the oscillation displacement $d_{ext}$ expressed by Formula (4) shown above, oscillation velocity $v_{ext}$ expressed by Formula (5) shown below can be calculated:

[Formula 5]

$$\boldsymbol{v}_{ext} = \tfrac{d}{dt} \boldsymbol{d}_{ext} \in \mathbb{C}^M \qquad (\,5\,)$$

Next, in step S15 illustrated in FIG. 12, the signal processing unit 10 extracts cardiac sound, RRI, and/or HRV, etc. from the results of processing in step S14. Step S15 includes several kinds of signal processing. A flowchart in FIG. 13 illustrates the signal processing performed in step S15 of FIG. 12 in more detail. The operation or processing illustrated in FIG. 13 may be performed by, for example, the calculation unit 14 of the signal processing unit 10. The operation or processing illustrated in FIG. 13 will now be further described below.

Upon the start of the operation in step S15 illustrated in FIG. 12, that is, the operation illustrated in FIG. 13, in step S21, the signal processing unit 10 performs processing for removing noise (denoising). Various methods may be used for the denoising performed in step S21. For example, in step S21, the signal processing unit 10 may perform the denoising of a time-series waveform using, for example, an empirical Bayes method, a wavelet method, or the like.

Next, in step S22, the signal processing unit 10 extracts a desired signal waveform (cardiac sound waveform) by using a discrete wavelet method such as a maximum overlap discrete Wavelet transform (MODWT).

The processing performed in step S21 and/or step S22 will now be further described.

In step S21, the signal processing unit 10 further performs preprocessing for removing unwanted noise on a signal velocity vector $v_{ext}$.

In step S21, the signal processing unit 10 may perform the denoising based on band limitation using an empirical Bayes method, a continuous wavelet, or the like. In step S21, the signal processing unit 10 may perform the denoising based on frequency subtraction using a noise profile on artifact noise, etc. arising from nonlinear processing from step S11 to step S14 in FIG. 12.

The signal processing unit 10 may perform multi-resolution analysis based on discrete wavelet transform by applying a wavelet waveform similar to a cardiac sound waveform to the preprocessed denoised signal. In this way, in step S22, the signal processing unit 10 extracts only a subspace in the level of multi-resolution analysis where cardiac sound of the person 200 who is the subject exists empirically. In this way, in step S22, the signal processing unit 10 may extract the cardiac sound of the person 200 who is the subject. Specifically, the signal processing unit 10 may use maximum overlap multi-resolution analysis (MODWT) or the like in order to enhance temporal resolution. A wavelet basis suitable for extraction of a heartbeat may be, for example, Symlet, Daubechies, and the like. The order of them may be set appropriately.

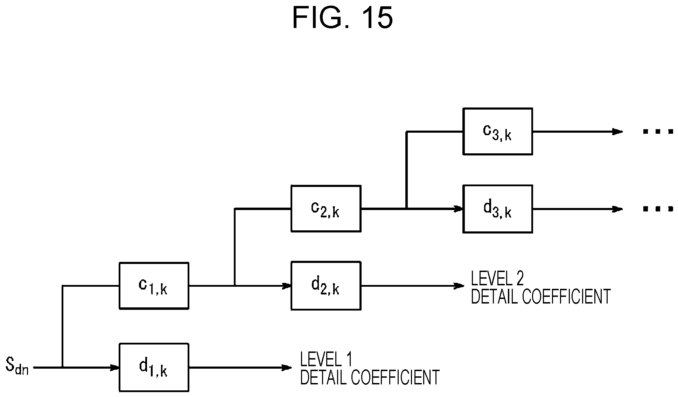

For example, multi-resolution analysis illustrated in FIG. 15 may be applied to the real part Re($v_{dn}$), where $v_{dn}$ denotes a signal obtained by performing the denoising of step S21 on the signal velocity vector $v_{ext}$. FIG. 15 is a diagram that conceptually illustrates multi-resolution analysis based on discrete wavelet transform. The signal processing unit 10 can obtain a cardiac sound waveform h through reconfiguration by limiting an empirically suitable level to $j_0 \in N$ in Formula (6) shown below.

[Formula 6]

$$h = \frac{1}{\sqrt{M}} \left[ \sum_{k=1}^{M-1} c_k \phi_{j_0,k} + \sum_{j=j_1}^{j_0} \sum_{k=1}^{M-1} d_{j,k} \psi_{j,k} \right] \in \mathbb{R}^M \qquad (6)$$

, where $\Phi_{j_0,k}$, $\Psi_{j,k}$ denote a vector of a scale function and a vector of a wavelet function respectively, and $c_k$, $d_{j,k}$ denote respective coefficients thereof.

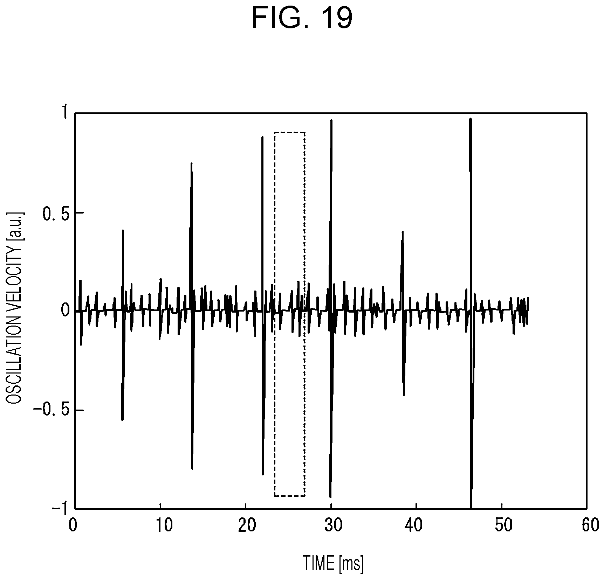

The cardiac sound waveform h obtained from Formula (6) shown above indicates the cardiac sound of the person 200 who is the subject with high precision (see FIGs. 19 and 20 to be described later). This result indicates that accuracy that is high enough for use for medical examination of cardiac sound and the like on an as-is basis can be expected.

Next, in step S23, the signal processing unit 10 detects the peaks of cardiac sound by using a scalogram. The signal processing unit 10 detects the power (level) of the peak at each point in time. The point in time where cardiac sound is positioned is detected as the position of the peak. In step S23, the signal processing unit 10 detects (extracts) the peaks in energy of the desired signal (cardiac sound waveform) having been extracted in step S22. Therefore, the signal processing unit 10 extracts an envelope waveform by using the scalogram through continuous wavelet transform and by performing rendering into one dimension thereof.

More specifically, in order to obtain the envelope waveform of the cardiac sound waveform h, the signal processing unit 10 obtains the scalogram through the continuous wavelet transform. The signal processing unit 10 obtains a one-dimension waveform by finding the maximum value in the frequency-axis direction at each point in time on the scalogram.

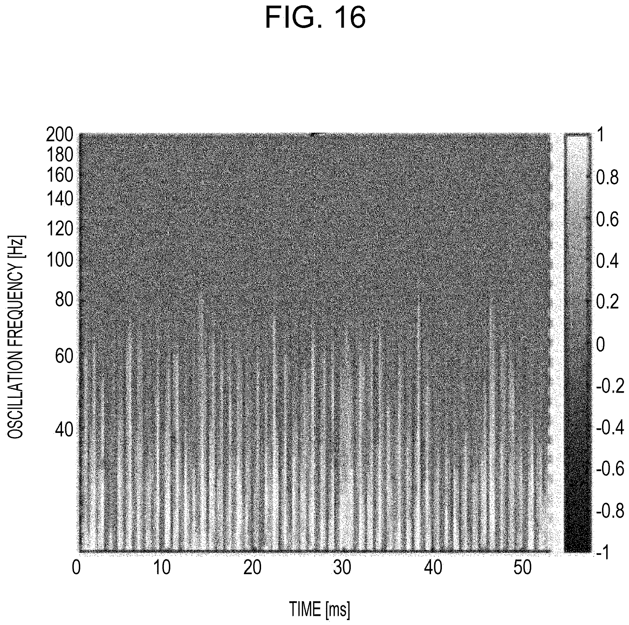

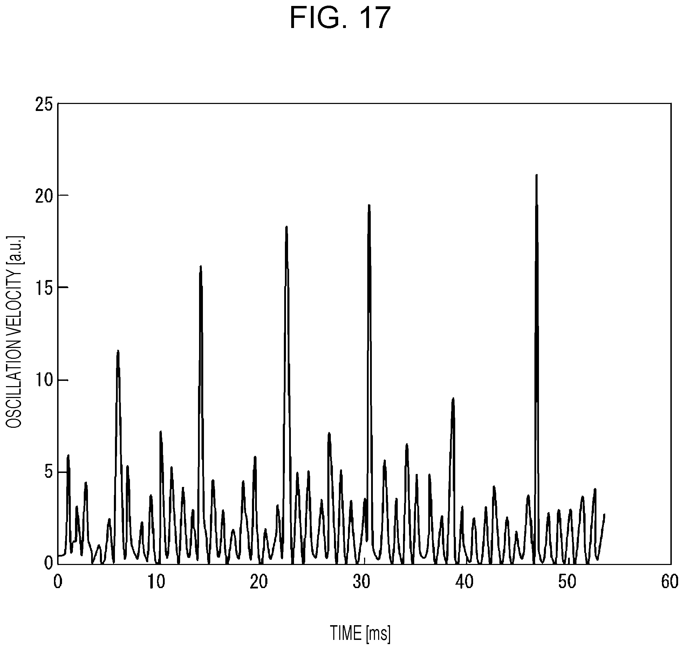

FIGs. 16 and 17 are diagrams for explaining the multi-resolution analysis based on the discrete wavelet transform. FIG. 16 is a diagram illustrating an example of the scalogram of the cardiac sound waveform h. FIG. 17 is a diagram illustrating an example of a one-dimension-rendered scalogram of the cardiac sound waveform h illustrated in FIG. 16.

When a matrix that represents absolute values of the scalogram illustrated in FIG. 16 is expressed as $S_h \in \mathbb{R}^{M \times L}$, the one-dimension-rendered scalogram illustrated in FIG. 17 can be calculated using a "for statement" expressed by Formulas (7) to (9) shown below: for

$$\text{for } m = 1 : M \qquad (7)$$

[Formula 7]

$$s_h(m) = \max_f S_h \ (\forall \ f, m) \in \mathbb{R}^M \qquad (8)$$

end (9)

In Formula (7) shown above, Sh(m) denotes the m-th element in the rendering, to one dimension, of the scalogram of the cardiac sound waveform h illustrated in FIG. 17, and f denotes each frequency of the scalogram.

Next, in step S24, the signal processing unit 10 controls the roughness of the peaks of the scalogram. In step S24, in order to appropriately calculate the peaks of the envelope waveform of the cardiac sound having been extracted in step S23, the signal processing unit 10 further removes high-frequency components by means of an appropriate range. Therefore, the signal processing unit 10 may adjust the smoothness of the envelope by performing discrete wavelet transform using a Haar wavelet or like.

The signal processing unit 10 may perform signal reconfiguration by using multi-resolution analysis based on discrete wavelet transform on a vector $s_h$ having been calculated as the envelope waveform of the cardiac sound for the purpose of adjusting its smoothness. A wavelet basis suitable to be used here may be a Haar wavelet. The concept of multi-resolution decomposition may be, for example, the same as or similar to that of FIG. 15. The formula for reconfiguration of multi-resolution decomposition may be, for example, the same as or similar to that of Formula (6) shown above. A reference sign $s_{h,rc}$ will be used hereinafter for the vector obtained by reconfiguring the vector $s_h$ having been calculated as the envelope waveform of the cardiac sound.

Next, in step S25, the signal processing unit 10 pinpoints the positions of a first cardiac sound and a second cardiac sound that have been made clear through the processing up to step S25. In step S25, the signal processing unit 10 may pickup the first cardiac sound S 1 and the second cardiac sound S2 in the cardiac sound and may perform pairing of the first cardiac sound S 1 and the second cardiac sound S2.

For example, as illustrated in FIG. 17, the vector $s_{h,rc}$ having been reconfigured as the envelope waveform of the cardiac sound has accuracy that is high enough for clearly indicating the first cardiac sound and the second cardiac sound. Therefore, in step S25, the signal processing unit 10 may perform pairing of the first cardiac sound S1 and the second cardiac sound S2 in order to measure the interval between the first cardiac sounds S1 accurately. To perform the pairing mentioned above, first, the signal processing unit 10 may perform peak detection for every peak of $s_{h,rc}$.

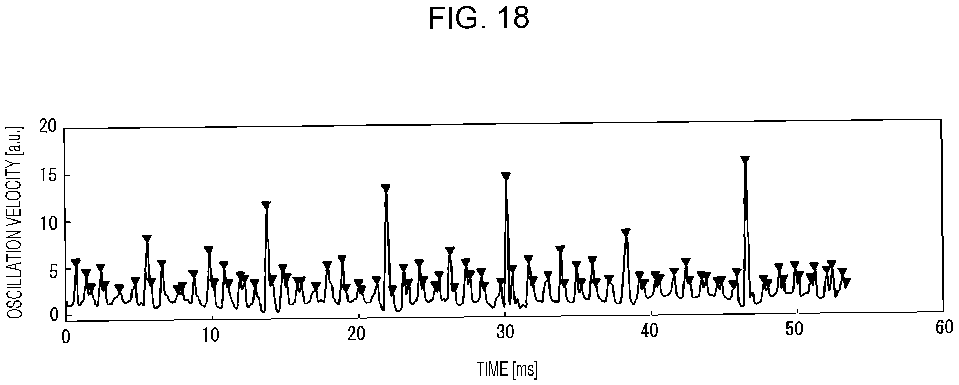

FIG. 18 is a diagram illustrating an example of execution of peak detection of a heartbeat envelope waveform. The signal processing unit 10 may create a row vector $l_{s1,s2} \in R^K$ of a time list by executing the peak detection illustrated in FIG. 18. A case where DBSCAN, which is a clustering algorithm, is used for a row vector $l_{s1,s2}$ will be described below. The signal processing unit 10 sets, as 1, minPts (the minimum number of neighboring data points other than the self), which is a parameter of DBACAN. The signal processing unit 10 measures $\varepsilon$ (distance between neighboring data points), which is a parameter of DBACAN, in terms of time. In this way, the signal processing unit 10 sets a standard time interval for the first cardiac sound S1 and the second cardiac sound S2 as a numerical value measured in terms of time to 0.2 to 0.4 seconds or so.

Through this processing, a pair of the first cardiac sound S1 and the second cardiac sound S2 is determined. Accordingly, the signal processing unit 10 adds a second row to the row vector $l_{s1,s2}$, and creates a list matrix $L_{s1,s2}$ by assigning its row cluster number (natural number of pair number 1 or greater) to the first cardiac sound S1 and by assigning a numerical value 0 to the second cardiac sound S2. By this means, the signal processing unit 10 is capable of, in the list matrix $L_{s1,s2}$, identify the first cardiac sound S1 clearly, and indicate its order clearly. When S1 is not paired in the row vector $l_{s1,s2}$ or in the list matrix $L_{s1,s2}$, the signal processing unit 10 may list the non-paired peak as S1.

Next, in step S26, the signal processing unit 10 calculates the heart rate interval RRI. In step S26, the signal processing unit 10 may calculate the RRI by calculating the interval between the peaks having been paired first in step S25, that is, the interval between the first cardiac sounds S1.

The signal processing unit 10 calculates the RRI by finding the interval between two adjacent S1 in the list matrix $L_{s1,s2}$. The RRI time-series data calculated here is expected to have outliers. The outlier can be generated due to, for example, a non-detection of the first cardiac sound S1, a mix-up between the first cardiac sound S1 and the second cardiac sound S2, and any other reason such as the influence of noise caused by a significant body motion, etc. Therefore, in one embodiment, the signal processing unit 10 may perform correction processing to deal with the above circumstances.

In step S26, the signal processing unit 10 may use a method based on Bayes' estimation such as a Kalman filter for the correction processing. Heartbeat response is nonlinear, and plant modeling is thus physically difficult. Therefore, in step S26, the signal processing unit 10 may perform correction processing based on a statistical method such as an unscented Kalman filter (UKF). On the other hand, a linear Kalman filter may be used approximatively, or an extended Kalman filter (EKF) may be used by modeling the transit of the heart rate interval by an empirical modeling approach.

The data obtained in step S26 may be RRI time-series data $s_{RRI}$.

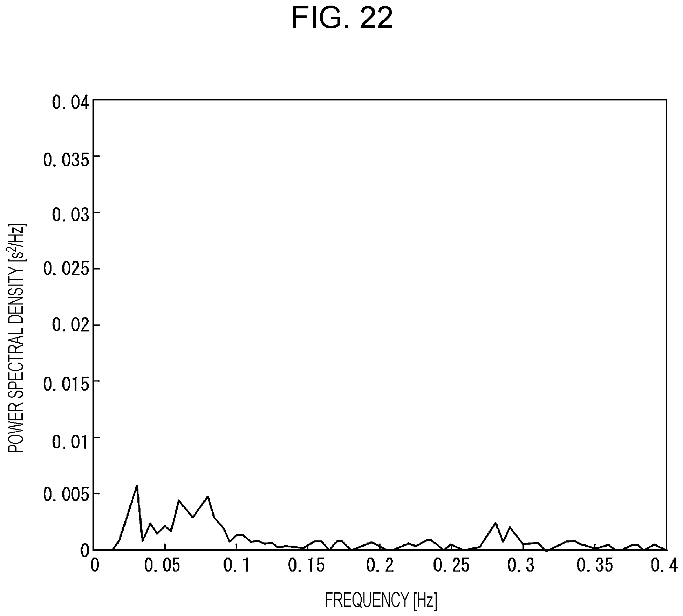

Next, in step S27, the signal processing unit 10 performs spectrum analysis. In step S27, the signal processing unit 10 may calculate a spectrogram of RRI (HRV). In step S27, the signal processing unit 10 may calculate the power spectral density of the time-series data of the RRI by using, for example, Welch's method or the like. Finally, the signal processing unit 10 may perform FFT calculation and power spectrum calculation for the $s_{RRI}$ in accordance with Welch's method or the like, that is, by means of a window function overlapping timewise. Through this processing, the signal processing unit 10 is capable of obtaining the power spectral density of the RRI (see FIG. 22 to be described later).

FIG. 19 is a diagram illustrating a time-series waveform of cardiac sound obtained by means of the electronic device 1 according to one embodiment. The waveform illustrated in FIG. 19 may be the cardiac sound waveform h obtained using Formula (6) described above. FIG. 20 is an enlarged view of a time span in a region enclosed by a broken-line frame in FIG. 19.

As illustrated in FIG. 20, the time-series waveform of cardiac sound obtained by means of the electronic device 1 according to one embodiment makes it possible to identify the first cardiac sound S1 and the second cardiac sound S2 clearly. In addition, as illustrated in FIG. 20, the time-series waveform of cardiac sound obtained by means of the electronic device 1 according to one embodiment makes it possible to clearly identify the heart rate interval RRI, which is calculated from the interval of the first cardiac sound S1 and the second cardiac sound S2, too. Though the RRI and the heart rate interval are not exactly identical to each other, they are believed to match approximatively. Therefore, in the present disclosure, the RRI and the heart rate interval are assumed to indicate the identical thing.

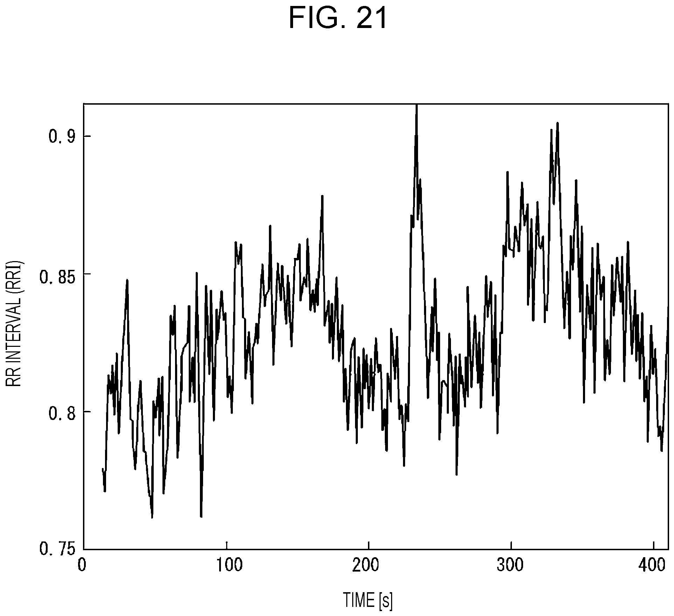

FIG. 21 is a diagram illustrating an example of the time-series waveform of the RRI illustrated in FIGs. 19 and 20. The result illustrated in FIG. 21 is calculated based on the cardiac sound extracted accurately in FIGS. 19 and 20. Therefore, the result illustrated in FIG. 21 has accuracy of some milliseconds.

FIG. 22 is a diagram illustrating the power spectral density of the RRI. FIG. 22 is a diagram illustrating an example of the power spectral density calculated using Welch's method from the time-series waveform of the RRI illustrated in FIG. 21. The electronic device 1 according to one embodiment makes it possible to calculate the power spectral density of the RRI on the basis of the RRI having accuracy of some milliseconds. Therefore, with the electronic device 1 according to one embodiment, the components from 0.15 Hz to 0.4 Hz, which is called HF of heartbeat PSD, can also be calculated accurately.

As explained above, the electronic device 1 according to one embodiment makes it possible to obtain, for example, a detailed cardiac sound waveform such as one illustrated in FIGs. 19 and 20, accurate RRI time-series data such as one illustrated in FIG. 21, and power spectral density (PSD) of a heart rate interval such as one illustrated in FIG. 22. The electronic device 1 according to one embodiment makes it possible to detect a heartbeat in a human body or the like with good accuracy through transmission and reception of a radio wave. Therefore, with the electronic device 1 according to one embodiment, weak oscillations such as those of a heartbeat in a human body or the like can be detected with good accuracy through transmission and reception of a radio wave such as, for example, a millimeter wave, which is expected to be useful in various fields.

In the examples illustrated in FIGs. 19 to 22, only the first cardiac sound S1 and the second cardiac sound S2 that are typically seen in healthy subjects have been described. However, with the electronic device 1 according to one embodiment, the same processing, or similar processing, can be performed also in a case of existence of a third cardiac sound and/or a fourth cardiac sound due to heartbeat abnormality.

(Other Embodiments)

Other embodiments will be described below.

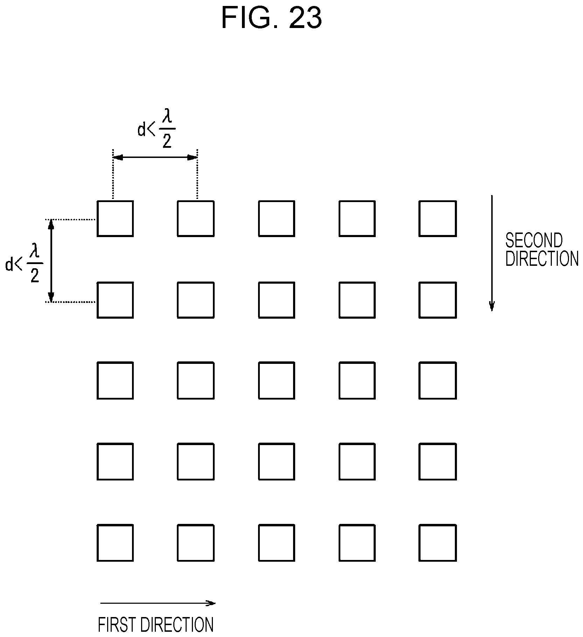

In one embodiment, the layout of the transmission antenna array 24 and/or the reception antenna array 31 of the electronic device 1 is not limited to the layout illustrated in FIG. 8. For example, in one embodiment, the reception antenna array 31 of the electronic device 1 may have a configuration illustrated in FIG. 23. FIG. 23 is a diagram illustrating an example of a URA (uniform rectangular array) reception antenna. By adopting the URA reception antenna such as one illustrated in FIG. 23, the direction-of-arrival estimation of two angles may be performed using the URA reception antenna alone, without changing the directivity by means of a beam former in the transmission antenna array 24.

In the electronic device 1 illustrated in FIG. 2, the signal processing unit 10 have been described as including functional units such as the heartbeat extraction unit 13 and the calculation unit 14. However, in one embodiment, the processing performed by the heartbeat extraction unit 13 and/or the calculation unit 14 may be performed by an external computer, an external processor, or the like.

In one embodiment, the processing in step S13 (SVD processing) illustrated in FIG. 12 may be replaced by any other subspace method.

In one embodiment, the processing in step S13 (SVD processing) illustrated in FIG. 12 may be omitted in a case where a large number can be set for the number of samples N of one chirp or in a case where other noise contamination can be eliminated by means of a hardware method or the like.

In one embodiment, the processing in step S24 illustrated in FIG. 13 may be omitted depending on a situation of implementation.

In one embodiment, the RRI may be obtained by calculating the interval between the second cardiac sounds S2 after the pairing of the first cardiac sound S1 and the second cardiac sound S2 in step S25 illustrated in FIG. 13.

As explained above, the electronic device 1 according to one embodiment detects weak oscillations such as those of a heartbeat by using, for example, a millimeter-wave sensor including a plurality of transmission antennas and a plurality of reception antennas. If a subject is not detected, the electronic device 1 according to one embodiment detects a body movement of the subject by performing a beamforming pattern of the transmission antennas while changing transmission phases of the antennas. On the other hand, if a body movement of the subject is detected, the electronic device 1 according to one embodiment detects a heartbeat by performing beamforming in a direction of the body movement. As described above, the electronic device 1 according to one embodiment automatically detects a direction of a human body, and thus can improve a signal quality. Therefore, the electronic device 1 according to one embodiment can improve a heartbeat detection accuracy and/or a heartbeat detection range. Therefore, the electronic device 1 according to one embodiment can detect a heartbeat of a human with high accuracy.

While the present disclosure has been described based on various drawings and embodiments, it is to be noted that a person skilled in the art can easily make various variations or changes based on the present disclosure. Therefore, it is to be noted that these variations or changes are within the scope of the present disclosure. For example, functions and the like included in each functional unit can be reconfigured without causing any logical contradiction. A plurality of functional units or the like may be combined into one or may be divided. Each embodiment according to the present disclosure described above is not limited to strict implementation in accordance with each description of the embodiment, and may be implemented by appropriately combining the features or omitting a part thereof. That is, based on the present disclosure, a person skilled in the art can make various variations and changes to the content of the present disclosure. Therefore, the scope of the present disclosure encompasses these variations and changes. For example, in each embodiment, each functional unit, each means, each step, or the like can be added to another embodiment or replaced with each functional unit, each means, each step, or the like in another embodiment without causing any logical contradiction. In each embodiment, a plurality of functional units, means, steps, or the like may be combined into one or may be divided. In addition, each embodiment according to the present disclosure described above is not limited to strict implementation in accordance with each description of the embodiment, and may be implemented by appropriately combining the features or omitting a part thereof.

The embodiments described above are not limited to implementation as the electronic device 1. For example, the embodiments described above may be implemented as a method for controlling a device such as the electronic device 1. For example, the embodiments described above may be implemented as a program to be run by a device such as the electronic device 1.

The electronic device 1 according to the above embodiments have been described as a device that includes components constituting a so-called radar sensor, such as the transmission antenna array 24 and the reception antenna array 31. However, the electronic device according to one embodiment may be implemented as a component such as, for example, the signal processing unit 10. In this case, for example, the signal processing unit 10 may have a function for processing signals dealt with by the transmission antenna array 24 and the reception antenna array 31.

REFERENCE SIGNS

| 1  | electronic device |
| 10 | signal processing unit |
| 11 | signal generation processing unit |
| 12 | reception signal processing unit |
| 13 | heartbeat extraction unit |
| 14 | calculation unit |
| 21 | transmission DAC |
| 22 | transmission circuit |
| 23 | millimeter-wave transmission circuit |
| 24 | transmission antenna array |
| 31 | reception antenna array |
| 32 | mixer |
| 33 | reception circuit |
| 34 | reception ADC |
| 50 | communication interface |
| 60 | external device |

**Claims**

1.  An electronic device, comprising:

    a transmission antenna configured to transmit a transmission wave;
    a reception antenna configured to receive a reflected wave, the reflected wave being the transmission wave having been reflected; and
    a signal processor configured to detect, based on a transmission signal transmitted as the transmission wave and a reception signal received as the reflected wave, oscillations arising from a heartbeat of a subject that reflects the transmission wave, wherein
    based on oscillation velocity obtained through oscillation component removal except for an oscillation component lying at a desired position from a result of performing a fast Fourier transform in a range direction and a velocity direction on the reception signal, the signal processor calculates the heartbeat of the subject.

2.  The electronic device according to claim 1, wherein
    the signal processor extracts a region where the subject is present by using a predetermined window function on a range-Doppler plane calculated by performing the fast Fourier transform in the range direction and the velocity direction on the reception signal.

3.  The electronic device according to claim 2, wherein
    the signal processor extracts the region where the subject is present by using, as the predetermined window function, at least one selected from the group consisting of a hanning window, a hamming window, and a Blackman-Harris window.

4.  The electronic device according to any of claims 1 to 3, wherein
    the signal processor extracts an element of the oscillations as cardiac sound of the subject by performing singular value decomposition on the result of performing the fast Fourier transform in the range direction and the velocity direction on the reception signal.

5.  The electronic device according to any of claims 1 to 4, wherein
    the signal processor extracts an element of the oscillations as cardiac sound of the subject by performing principal component analysis of the result of performing the fast Fourier transform in the range direction and the velocity direction on the reception signal.

6.  The electronic device according to any of claims 1 to 5, wherein
    the signal processor performs processing to denoise a time-series waveform by using an empirical Bayes method or a wavelet method.

7.  The electronic device according to any of claims 1 to 6, wherein
    the signal processor extracts a waveform of cardiac sound of the subject by using a discrete wavelet method.

8.  The electronic device according to claim 7, wherein
    the signal processor performs multi-resolution analysis based on a discrete wavelet transform by using a wavelet waveform similar to a waveform of the cardiac sound of the subj ect.

9.  The electronic device according to claim 8, wherein
    based on a scalogram obtained through a continuous wavelet transform, the signal processor obtains an envelope waveform of the cardiac sound of the subject.

10. The electronic device according to claim 9, wherein
    the signal processor obtains the envelope waveform of the cardiac sound of the subject by identifying a first cardiac sound and a second cardiac sound of the subject.

11. The electronic device according to claim 10, wherein
    the signal processor obtains the envelope waveform of the cardiac sound of the subject by identifying two first cardiac sounds adjacent to each other included in the cardiac sound of the subject.

12. The electronic device according to any of claims 8 to 11, wherein
    the signal processor corrects an interval in the heartbeat of the subject by using a Bayes' estimation method.

13. An electronic device configured to detect, based on a transmission signal transmitted as a transmission wave from a transmission antenna and a reception signal received via a reception antenna as a reflected wave, the reflected wave being the transmission wave having been reflected, oscillations arising from a heartbeat of a subject that reflects the transmission wave, wherein
    the heartbeat of the subject is calculated based on oscillation velocity obtained through oscillation component removal except for an oscillation component lying at a desired position from a result of performing a fast Fourier transform in a range direction and a velocity direction on the reception signal.

14. A method for controlling an electronic device, comprising:

    transmitting a transmission wave from a transmission antenna;
    receiving, via a reception antenna, a reflected wave, the reflected wave being the transmission wave having been reflected;
    detecting, based on a transmission signal transmitted as the transmission wave and a reception signal received as the reflected wave, oscillations arising from a heartbeat of a subject that reflects the transmission wave; and
    calculating the heartbeat of the subject, based on oscillation velocity obtained through oscillation component removal except for an oscillation component lying at a desired position from a result of performing a fast Fourier transform in a range direction and a velocity direction on the reception signal.

**15.** A program for causing an electronic device to execute:

transmitting a transmission wave from a transmission antenna;
receiving, via a reception antenna, a reflected wave, the reflected wave being the transmission wave having been reflected;
detecting, based on a transmission signal transmitted as the transmission wave and a reception signal received as the reflected wave, oscillations arising from a heartbeat of a subject that reflects the transmission wave; and
calculating the heartbeat of the subject, based on oscillation velocity obtained through oscillation component removal except for an oscillation component lying at a desired position from a result of performing a fast Fourier transform in a range direction and a velocity direction on the reception signal.

# FIG. 1

**FIG. 2**

FIG. 3

# FIG. 4

SUB-FRAME 1

c1
c2
c3
c4
⋮
cn

⋮

SUB-FRAME N

c1
c2
c3
c4
⋮
cn

# FIG. 5

# FIG. 6

FIG. 7

EP 4 390 455 A1

# FIG. 8

$$d_{1,t} < \frac{\lambda}{2}$$

24

$$d_{2,t} < \frac{\lambda}{2}$$

$$d_{1,s} < \frac{\lambda}{2}$$

31

$$d_{2,s} < \frac{\lambda}{2}$$

SECOND DIRECTION

FIRST DIRECTION

# FIG. 9

FIG. 10

EP 4 390 455 A1

# FIG. 11

```
        ( START )
            |
            v
+---------------------------------+
|  PROCESSING OF RECEPTION SIGNAL |  ~ S110
+---------------------------------+
            |
            v
+---------------------------------+
|  EXTRACTION OF OSCILLATION SOURCE |  ~ S120
+---------------------------------+
            |
            v
+-----------------------------------+
| CONVERSION INTO OSCILLATION WAVEFORM |  ~ S130
+-----------------------------------+
            |
            v
+---------------------------------+
|  EXTRACTION OF OSCILLATION DATA |  ~ S140
+---------------------------------+
            |
            v
+---------------------------------+
|  DETECTION OF PEAKS OF BEATING  |  ~ S150
+---------------------------------+
            |
            v
+---------------------------------+
|       CALCULATION OF RRI        |  ~ S160
+---------------------------------+
            |
            v
+---------------------------------+
|       ANALYSIS OF SPECTRUM      |  ~ S170
+---------------------------------+
            |
            v
        (  END  )
```

# FIG. 12

```
        ┌─────────────┐
        │    START    │
        └─────────────┘
               │
               ▼
┌──────────────────────────────────────┐
│     PROCESSING OF RECEPTION SIGNAL    │ ⟋ S11
└──────────────────────────────────────┘
               │
               ▼
┌──────────────────────────────────────┐
│     EXTRACTION OF OSCILLATION SOURCE  │ ⟋ S12
└──────────────────────────────────────┘
               │
               ▼
┌──────────────────────────────────────┐
│   EXTRACTION OF ELEMENTS OF OSCILLATIONS │ ⟋ S13
└──────────────────────────────────────┘
               │
               ▼
┌──────────────────────────────────────┐
│   CONVERSION INTO OSCILLATION WAVEFORM │ ⟋ S14
└──────────────────────────────────────┘
               │
               ▼
┌──────────────────────────────────────┐
│     EXTRACTION OF CARDIAC SOUND, ETC.  │ ⟋ S15
└──────────────────────────────────────┘
               │
               ▼
        ┌─────────────┐
        │     END     │
        └─────────────┘
```

# FIG. 13

START

REMOVAL OF NOISE — S21

EXTRACTION OF CARDIAC SOUND — S22

DETECTION OF PEAKS USING SCALOGRAM — S23

CONTROL OF PEAK ROUGHNESS — S24

PINPOINTING OF POSITIONS OF FIRST AND SECOND CARDIAC SOUNDS — S25

CALCULATION OF RRI — S26

ANALYSIS OF SPECTRUM — S27

END

FIG. 14

# FIG. 15

# FIG. 16

# FIG. 17

FIG. 18

EP 4 390 455 A1

# FIG. 19

# FIG. 20

# FIG. 21

# FIG. 22

# FIG. 23

$$d < \frac{\lambda}{2}$$

$$d < \frac{\lambda}{2}$$

SECOND
DIRECTION

FIRST DIRECTION

## INTERNATIONAL SEARCH REPORT

| | International application No. |
|---|---|
| | **PCT/JP2022/029856** |

### A. CLASSIFICATION OF SUBJECT MATTER

***G01S 13/34***(2006.01)i; ***A61B 5/0245***(2006.01)i
FI: G01S13/34; A61B5/0245 Z

According to International Patent Classification (IPC) or to both national classification and IPC

### B. FIELDS SEARCHED

Minimum documentation searched (classification system followed by classification symbols)

G01S 7/00 - G01S 7/42, G01S 13/00 - G01S 13/95, A61B 5/02 - A61B 5/03

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2021-001735 A (ASAHI KASEI ELECTRONICS CO., LTD.) 07 January 2021 (2021-01-07) paragraphs [0021]-[0028], [0042]-[0066], [0078]-[0106], [0120], [0133], [0143]-[0168], fig. 5-9B, 14, 15 | 1-3, 13-15 |
| Y | | 4-12 |
| Y | CN 112401856 A (WUHAN FENGHUO KAIZHUO TECHNOLOGY CO., LTD.) 26 February 2021 (2021-02-26) paragraphs [0026], [0031], [0045]-[0051] | 4-5 |
| Y | JUNG, Marie et al. Non-contact Blood Pressure Estimation Using a 300 GHz Continuous Wave Radar and Machine Learning Models. 2021 IEEE International Symposium on Medical Measurements and Applications (MeMeA) [online]. 12 July 2021, p. 6, <DOI: 10.1109/ MeMeA52024.2021.9478734> particularly, sections II. C, III. A | 6-12 |
| Y | WO 2016/042758 A1 (PANASONIC INTELLECTUAL PROPERTY MANAGEMENT CO., LTD.) 24 March 2016 (2016-03-24) paragraphs [0104]-[0109], [0125], [0126], fig. 16B | 9-11 |

☑ Further documents are listed in the continuation of Box C.    ☑ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **31 August 2022** | **13 September 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| | International application No. |
|---|---|
| | **PCT/JP2022/029856** |

### C. DOCUMENTS CONSIDERED TO BE RELEVANT

| Category* | Citation of document, with indication, where appropriate, of the relevant passages | Relevant to claim No. |
|---|---|---|
| Y | WO 2019/049667 A1 (MURAKAMI CORP.) 14 March 2019 (2019-03-14) paragraph [0054] | 12 |
| A | WO 2021/151812 A1 (IEE INTERNATIONAL ELECTRONICS & ENGINEERING S.A.) 05 August 2021 (2021-08-05) paragraphs [0036]-[0063], fig. 1-8 | 1-15 |
| P, A | CN 113261925 A (SHANDONG NORMAL UNIVERSITY) 17 August 2021 (2021-08-17) paragraphs [0030]-[0071], fig. 1-5 | 1-15 |

Form PCT/ISA/210 (second sheet) (January 2015)

INTERNATIONAL SEARCH REPORT
Information on patent family members

| International application No. |
|---|
| **PCT/JP2022/029856** |

| Patent document cited in search report | | | Publication date (day/month/year) | Patent family member(s) | | | Publication date (day/month/year) |
|---|---|---|---|---|---|---|---|
| JP | 2021-001735 | A | 07 January 2021 | (Family: none) | | | |
| CN | 112401856 | A | 26 February 2021 | (Family: none) | | | |
| WO | 2016/042758 | A1 | 24 March 2016 | JP | 2016-059718 | A | |
| | | | | CN | 106572815 | A | |
| | | | | KR | 10-2017-0057226 | A | |
| WO | 2019/049667 | A1 | 14 March 2019 | US | 2020/0245875 | A1 | |
| | | | | paragraph [0085] | | | |
| | | | | CN | 111050640 | A | |
| WO | 2021/151812 | A1 | 05 August 2021 | LU | 101622 | B1 | |
| CN | 113261925 | A | 17 August 2021 | (Family: none) | | | |

Form PCT/ISA/210 (patent family annex) (January 2015)

48

**EP 4 390 455 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021132423 A
- JP 2002071825 A
- JP 2021032880 A